# EUROPEAN PATENT APPLICATION

(11) **EP 4 378 754 A1**
(43) Date of publication of application: **05.06.2024**
(21) Application number: 22849251.8
(22) Date of filing: 12.07.2022
(51) Int. Cl.: B60N 2/90, A47C 7/62, A47C 7/74, A61B 5/107, A61B 5/113, B60N 2/56, G01L 5/00

(54) **INFERENCE DEVICE, CONTROL DEVICE, INFERENCE METHOD AND INFERENCE PROGRAM**

(30) Priority: 26.07.2021 JP 2021122012; 26.07.2021 JP 2021122013; 26.07.2021 JP 2021122014; 26.07.2021 JP 2021122021; 26.07.2021 JP 2021122022
(71) Applicant: BRIDGESTONE CORPORATION, Chuo-ku Tokyo 104-8340 (JP)
(72) Inventor: KITANO, Hajime, Tokyo 104-8340 (JP); WAKAO, Yasumichi, Tokyo 104-8340 (JP); SHINOHARA, Toshimitsu, Tokyo 104-8340 (JP); EBE, Kazushige, Tokyo 104-8340 (JP); ONIKI, Yoshihiko, Tokyo 104-8340 (JP)
(74) Representative: Marks & Clerk LLP
(86) International application number: PCT/JP2022/027482
(87) International publication number: WO 2023/008187

(57) **Abstract**

An inference device detects, with a detection section, an electrical characteristic between plural predetermined detection points on a vehicle seat provided with a soft material that is conductive and that exhibits a change in an electrical characteristic according to a change in pressure imparted. An inference section employs a learning model to infer a posture state of an occupant of the vehicle seat from the electrical characteristic of the vehicle seat. The electrical characteristic when pressure has been imparted to the vehicle seat and the posture state information indicating the posture state of the occupant when imparting pressure to the vehicle seat are employed as training data for a learning model trained so as to be input with the electrical characteristic and output the posture state information, and trained so as to be input with the electrical characteristic and output the posture state of the occupant corresponding to the input electrical characteristic.

## Description

### Technical Field

The present disclosure relates to an inference device, a control device, an inference method, and an inference program.

### Background Art

Hitherto, in order to identify a posture of an occupant seated in a vehicle seat, a shape change occurring in the vehicle seat is detected, and the posture of the occupant is inferred using this detection result. An aspect of detecting the shape change occurring in the vehicle seat is that it is difficult to detect deformation without impeding deformation of the vehicle seat. Moreover, a strain sensor employed to detect a rigid body such as a metal deformation or the like is difficult to utilize in a vehicle seat, and so there is a demand for a specialized detection device for detecting deformation of a vehicle seat. For example, technology is known for measuring displacement and vibration of a physical body using a camera, for acquiring a deformation image, and for extracting a deformation amount (see for example International Publication (WO) No. 2017-029905). Moreover, technology related to a soft touch sensor for inferring a deformation amount from an amount of light transmission is also known (see, for example, Japanese Patent Application Laid-Open (JP-A) No. 2013-101096).

Moreover, with respect to an aspect of maintaining an appropriate state for a posture of an occupant seated in a vehicle seat, technology is known for detecting the body pressure of an occupant seated in the vehicle seat of an automobile using a body pressure sensor, and driving a support member provided in a seatback, seat cushion, or the like according to the detected body pressure so as to maintain an appropriate distribution state of body pressure distribution when seated (see, for example, JP-A 2019-137286).

### SUMMARY OF INVENTION

### Technical Problem

However, with respect to an aspect of detecting shape change occurring in a vehicle seat, when detecting a deformation amount such as a displacement or the like of a physical body using a camera and image analysis method, a system that includes a camera, image analyzer, and the like is not preferable due to being bulky and leading to the device becoming more bulky. Moreover, measurement is not able to be performed of obscured portions not imageable by a camera with an optical method utilizing a camera. This means there is room for improvement in detecting deformation of a vehicle seat.

Moreover, with respect to an aspect of maintaining an appropriate state for the posture of an occupant seated in a vehicle seat, when employing a method of detecting vibration using a sensor such as a piezoelectric element or the like, there is a concern that the sensor itself might impede deformation of the vehicle seat. Moreover, there is also a concern that a seated state causing deformation of a vehicle seat might not be reflected in vibration detection, and there is room for improvement in inferring a seated state. In addition, an uncomfortable feeling is generated and the ride comfort is changed due to the occupant sitting on the sensor, and this leads to the inconvenience of not being able to perform both measurement and ride comfort evaluation at the same time.

An object of the present disclosure is to provide an inference device, a control device, an inference method, and an inference program that are capable of utilizing an electrical characteristic of a vehicle seat provided with a conductive soft material to infer posture state information indicating a posture state of an occupant of a vehicle seat, without using a specialized detection device.

### Solution to Problem

In order to achieve the above objective, a first aspect is an inference device including a detection section that, for a vehicle seat provided with a soft material that is conductive and exhibits a change in an electrical characteristic according to a change in pressure imparted, detects an electrical characteristic between plural predetermined detection points on the soft material of the vehicle seat, and an inference section that employs as training data a time series of the electrical characteristic when pressure has been imparted to the soft material and posture state information indicating a posture state of an occupant of the vehicle seat when imparting pressure to the soft material, that inputs the time series of the electrical characteristic detected by the detection section to a learning model trained to use the time series of the electrical characteristic as input so as to output the posture state information, and that infers posture state information indicating a posture state of the occupant corresponding to the input time series of the electrical characteristic.

A second aspect is the inference device of the first aspect, wherein the electrical characteristic is volume resistivity, the vehicle seat includes at least one out of a seat cushion, a seatback, a headrest, or an armrest, the posture state includes a seated state of an occupant of the vehicle seat, and the learning model is trained so as to output, as the posture state information, information indicating a seated state of the occupant corresponding to the detected electrical characteristic.

A third aspect is the inference device of the second aspect, wherein the vehicle seat includes a material imparted with conductivity to at least one part of a urethane member of a structure including a skeleton of at least one out of a fiber form or a mesh form or a structure having plural minute air bubbles inside.

A fourth aspect is the inference device of the second aspect or the third aspect, wherein the seated state includes a state related to breathing of the occupant of the vehicle seat, a state related to a sitting-style action of the occupant, and a state related to reclining of the vehicle seat, and the learning model is trained so as to output, as the posture state information, information indicating a state corresponding to the detected electrical characteristic that is at least one out of a state related to breathing of the occupant, a state related to a sitting-style action of the occupant, or a state related to reclining of the vehicle seat.

A fifth aspect is the inference device of any one of the first aspect to the fourth aspect, wherein the learning model includes a model that employs the soft material as a reservoir and that is generated by being trained with a network using reservoir computing using the reservoir.

A sixth aspect is the inference device of any one of the first aspect to the fifth aspect, wherein the soft material is a soft material that has conductivity and that has an electrical characteristic that changes according to change in imparted pressure and moisture, the posture state information is a time series of electrical characteristic when pressure and moisture is imparted to the soft material, and posture state information indicating a posture state in the presence of water of the occupant of the vehicle seat when imparting pressure and moisture to the soft material, and the inference section infers posture state information indicating a posture state in the presence of water of the occupant.

A seventh aspect is the inference device of the sixth aspect, wherein the posture state in the presence of water of the occupant includes a posture state in the presence of sweat in a seated state of the occupant.

An eighth aspect is the inference device of the sixth aspect or the seventh aspect, wherein the posture state includes a seated state in the presence of water of the occupant of the vehicle seat, and the learning model is trained so as to output, as the posture state information, information indicating a seated state in the presence of water of the occupant corresponding to the detected electrical characteristic.

A ninth aspect is the inference device of any one of the first aspect to the fifth aspect, wherein the posture state information is a time series of electrical characteristic when pressure is imparted to the soft material and posture state information indicating a posture state accompanying movement of an occupant of the vehicle seat when imparting pressure to the soft material, and the inference section infers posture state information indicating a posture state accompanying movement of the occupant.

A tenth aspect is the inference device of the ninth aspect, wherein the posture state includes a seated state accompanying movement of the occupant on the vehicle seat, and the learning model is trained so as to output, as the posture state information, information indicating a seated state accompanying movement of the occupant corresponding to the detected electrical characteristic.

An eleventh aspect is a control device including the inference device of any one of the sixth aspect to the eighth aspect, and a control section that employs the posture state information inferred by the inference section to control an environment conditioning device including at least one out of a temperature adjustment device of the vehicle seat or a conditioning device of a vehicle cabin installed with the vehicle seat.

A twelfth aspect is the control device of the eleventh aspect, wherein the vehicle seat includes at least one out of a seat cushion, a seatback, a headrest, or an armrest, the posture state includes a seated state in the presence of water of the occupant of the vehicle seat, and the control section controls the environment conditioning device using the posture state information inferred by the inference section.

A thirteenth aspect is a control device including the inference device of the ninth aspect or the tenth aspect, and a control section that controls a vehicle device configuring a vehicle installed with the vehicle seat using the posture state information inferred by the inference section.

A fourteenth aspect is the control device of the thirteenth aspect, wherein the inference section infers a state of an occupant as a seated state accompanying movement, and the control section controls the vehicle device based on the state of the occupant.

A fifteenth aspect is the control device of the fourteenth aspect, wherein the state of the occupant is a state related to the mind of an occupant, and the control section controls a seat drive device as the vehicle device such that a position of the vehicle seat is a position according to the state related to the mind of the occupant.

A sixteenth aspect is the control device of the fourteenth aspect, wherein the state of the occupant is a state related to the mind of an occupant, and the control section outputs an audio message according to the state related to the mind of the occupant from an audio output device as the vehicle device.

A seventeenth aspect is the control device of the fourteenth aspect, wherein the state of the occupant is a state related to a sitting-style action, and the control section controls a seat drive device as the vehicle device such that a position of the vehicle seat is a position according to the sitting-style action.

An eighteenth aspect is an inference method including a computer acquiring, for a vehicle seat provided with a soft material that is conductive and exhibits a change in an electrical characteristic according to a change in pressure imparted, an electrical characteristic between plural predetermined detection points on the soft material of the vehicle seat from a detection section that detects the electrical characteristic, the computer employing as training data a time series of the electrical characteristic when pressure has been imparted to the soft material and posture state information indicating a posture state of an occupant of the vehicle seat when imparting pressure to the soft material, inputting the time series of the electrical characteristic detected by the detection section to a learning model trained to use the time series of the electrical characteristic as input so as to output the posture state information, and the computer inferring posture state information indicating a posture state of the occupant corresponding to the input time series of the electrical characteristic.

A nineteenth aspect is an inference program that causes a computer to execute processing. The processing includes acquiring, for a vehicle seat provided with a soft material that is conductive and exhibits a change in an electrical characteristic according to a change in pressure imparted, an electrical characteristic between plural predetermined detection points on the soft material of the vehicle seat from a detection section that detects the electrical characteristic, and employing as training data a time series of the electrical characteristic when pressure has been imparted to the soft material and posture state information indicating a posture state of an occupant of the vehicle seat when imparting pressure to the soft material, inputting the time series of the electrical characteristic detected by the detection section to a learning model trained to use time series of the electrical characteristic as input so as to output the posture state information, and inferring posture state information indicating a posture state of the occupant corresponding to the input time series of the electrical characteristic.

### Advantageous Effect

The present disclosure exhibits the advantageous effect of being able to utilize an electrical characteristic of a vehicle seat provided with a conductive soft material to infer posture state information indicating a posture state of an occupant of the vehicle seat without using a specialized detection device.

### BRIEF DESCRIPTION OF DRAWINGS

Fig. 1 is a diagram illustrating an example of a configuration of a posture state inference device according to a first exemplary embodiment.
Fig. 2 is a diagram related to a vehicle seat according to the first exemplary embodiment.
Fig. 3 is a diagram related to detection points on a conductive member according to the first exemplary embodiment.
Fig. 4 is a diagram related to a conductive member according to the first exemplary embodiment.
Fig. 5 is a diagram related to a conductive member according to the first exemplary embodiment.
Fig. 6 is a diagram related to a conductive member according to the first exemplary embodiment.
Fig. 7 is a diagram related to training processing according to the first exemplary embodiment.
Fig. 8 is a flowchart illustrating an example of training data collection processing according to the first exemplary embodiment.
Fig. 9 is a diagram related to training processing in a training processing section according to the first exemplary embodiment.
Fig. 10 is a flowchart illustrating an example of a flow of training processing according to the first exemplary embodiment.
Fig. 11 is a diagram related to training processing in a training processing section according to the first exemplary embodiment.
Fig. 12 is a diagram illustrating an example of a configuration of a posture state inference device according to the first exemplary embodiment.
Fig. 13 is a flowchart illustrating an example of a flow of inference processing according to the first exemplary embodiment.
Fig. 14 is a diagram illustrating a characteristic related to a vehicle seat according to the first exemplary embodiment.
Fig. 15 is a diagram illustrating a characteristic related to a vehicle seat according to the first exemplary embodiment.
Fig. 16 is a diagram related to a conductive member according to the first exemplary embodiment.
Fig. 17 is a diagram illustrating an example of a configuration of a posture state inference device according to a second exemplary embodiment.
Fig. 18 is a diagram related to training processing according to the second exemplary embodiment.
Fig. 19 is a flowchart illustrating an example of training data collection processing according to the second exemplary embodiment.
Fig. 20 is a diagram illustrating a characteristic related to a vehicle seat according to the second exemplary embodiment.
Fig. 21 is a diagram illustrating a characteristic related to a vehicle seat according to the second exemplary embodiment.
Fig. 22 is a diagram related to training processing in a training processing section according to the second exemplary embodiment.
Fig. 23 is a flowchart illustrating an example of a flow of training processing according to the second exemplary embodiment.
Fig. 24 is a diagram related to training processing in a training processing section according to the second exemplary embodiment.
Fig. 25 is a flowchart illustrating an example of a flow of inference processing according to the second exemplary embodiment.
Fig. 26 is a diagram illustrating an example of a configuration of a control device according to a third exemplary embodiment.
Fig. 27 is a flowchart illustrating an example of a flow of inference/control processing according to the third exemplary embodiment.
Fig. 28 is a diagram illustrating an example of a configuration of a posture state inference device according to a fourth exemplary embodiment.
Fig. 29 is a diagram related to training processing according to the fourth exemplary embodiment.
Fig. 30 is a flowchart illustrating an example of training data collection processing according to the fourth exemplary embodiment.
Fig. 31 is a diagram related to training processing in a training processing section according to the fourth exemplary embodiment.
Fig. 32 is a flowchart illustrating an example of a flow of training processing according to the fourth exemplary embodiment.
Fig. 33 is a diagram related to training processing in a training processing section according to the fourth exemplary embodiment.
Fig. 34 is a flowchart illustrating an example of a flow of inference processing according to the fourth exemplary embodiment.
Fig. 35 is a diagram illustrating a characteristic related to a vehicle seat according to the fourth exemplary embodiment.
Fig. 36 is a diagram illustrating a characteristic related to a vehicle seat according to the fourth exemplary embodiment.
Fig. 37 is a diagram illustrating a characteristic related to a vehicle seat according to the fourth exemplary embodiment.
Fig. 38 is a diagram illustrating an example of a configuration of a control device according to a fifth exemplary embodiment.
Fig. 39 is a flowchart illustrating an example of a flow of inference/control processing according to the fifth exemplary embodiment.

### DESCRIPTION OF EMBODIMENTS

Detailed explanation follows regarding exemplary embodiments to implement technology disclosed herein, with reference to the drawings.

Note that the same reference numerals will be appended throughout the drawings to configuration elements and processing performing the same operation/function, and duplicate explanation thereof will sometimes be omitted as appropriate. Moreover, the present disclosure is not limited to any of the exemplary embodiments described below, and appropriate modifications may be implemented thereto within a range of the object of the present disclosure. Moreover, although in the present disclosure description follows regarding inference of a physical quantity for a member that mainly undergoes nonlinear deformation, obviously the present disclosure is applicable to inference of a physical quantity for a member that undergoes linear deformation.

### First Exemplary Embodiment

In the present disclosure "vehicle seat" refers, as an example, to a seat in a vehicle such as a car for an occupant to sit on, such as a driver seat, front passenger seat, rear seat of a second row or further rearward row, and encompasses a seat cushion serving as a seating face section for the occupant to sit on, a seatback serving as a backrest section supporting the back of the occupant, a headrest for supporting the head of the occupant, and an armrest for the occupant to rest an arm on. The "vehicle seat" encompasses a material deformable such that at least part of the vehicle seat flexes or the like when imparted with an external force. An example of an external force is pressure serving as an impetus imparted to the vehicle seat. The "posture state of the occupant seated in the vehicle seat" encompasses a state of the occupant imparting pressure to a soft material to deform the soft material, and includes a behavior of the occupant such as a posture, movement, or the like of the occupant seated in the vehicle seat. The posture state is a seated state of the occupant seated on the vehicle seat.

"Soft material" in the present disclosure encompasses a material that is deformable such that at least one part flexes or the like when imparted with an external force, and includes a soft elastic body such as a rubber material or the like, a structural body including a skeleton of at least one out of a fiber form or a mesh form, and a structural body with plural minute air bubbles distributed inside. An example of external force is pressure. A polymer material such as a urethane material or the like may be given as an example of a structural body including a skeleton of at least one out of a fiber form or a mesh form, and a structural body having plural minute air bubbles distributed inside. A "soft material imparted with conductivity" encompasses a conductive material, and includes a material having a conductive member appended to a soft material in order to impart conductivity, and also a material including a conductive soft material. Moreover, a soft material imparted with conductivity includes functionality in which an electrical characteristic changes according to deformation. Note that a pressure value indicating an impetus (hereafter referred to as a pressure impetus) from pressure imparted to a soft material may be given as an example of a physical quantity exhibiting a function of an electrical characteristic changing according to deformation. The soft material deforms according to external force arising from an occupant in a seated state or the like, for example according to a distribution of pressure impetus. An electrical resistivity may be given as an example of a physical quantity representing an electrical characteristic that changes according to deformation. Moreover, other examples thereof include a voltage value or a current value. The electrical resistivity may be captured as a volume resistivity of the soft material.

By being imparted with conductivity the soft material exhibits an electrical characteristic according to deformation by pressure. Namely, the soft material imparted with conductivity includes electrical pathways linking in a complex manner, with the electrical pathways elongating/compressing and expanding/contracting according to deformation. Moreover, sometimes the electrical pathways exhibit a behavior of being temporarily disconnected, or a behavior of connections different to previously arising. Thus the soft material exhibits a behavior of having a different electrical characteristic between positions separated by a specific distance (for example positions of detection points where electrodes are disposed) according to a magnitude and distribution of force (for example pressure impetus) imparted. This means that the electrical characteristic changes according to the size and distribution of force (for example pressure impetus) imparted to the soft material. Note that due to employing the soft material imparted with conductivity, there is no need to provide detection points such as electrodes or the like at all sites where pressure is imparted to the soft material by a physical body such as a person. Provide detection points such as electrodes or the like to at least two freely selected sites sandwiching a site where pressure is imparted to the soft material is sufficient.

The inference device of the present disclosure employs a pre-trained learning model to infer a posture state of an occupant seated on a vehicle seat from an electrical characteristic of a conductive soft material provided to the vehicle seat. The soft material may be disposed on the vehicle seat. As training data, the learning model employs a time series of an electrical characteristic when pressure has been imparted to the conductive soft material, and posture state information indicating the posture state of the occupant seated in the vehicle seat when pressure has been imparted to the soft material. The learning model is trained so as to be input with an electrical characteristic time series and to output posture state information indicating the posture state of the occupant seated on the vehicle seat corresponding to this electrical characteristic time series.

In the following explanation, a case is explained in which the vehicle seat employs a vehicle seat disposed with a seat member having a conductive material either impregnated or blended into all or at least one part of a urethane member (hereafter referred to as conductive urethane) serving as the conductive soft material. Note that the conductive urethane is provided to at least one out of a seat cushion, seatback, headrest, or armrest. Moreover, the conductive urethane may be discretely provided to the seat cushion, seatback, headrest, or armrest, or may be integrally provided thereto. Namely, at least one conductive urethane is provided to at least one sub-range of a range contacted by the occupant when seated on the vehicle seat. A thickness of the conductive urethane is, for example, preferably 1 mm or greater. Moreover, a volume resistivity of the conductive urethane is preferably, for example, 10⁷ Ω.cm or less. Moreover, a value (pressure value) indicating a pressure impetus imparted to the vehicle seat is employed as a physical quantity to deform the conductive urethane, and a seated state of the occupant seated on the vehicle seat is employed as the posture state. Moreover, description follows regarding a case in which an electrical resistivity of the conductive urethane is employed as the physical quantity that changes according to pressure impetus.

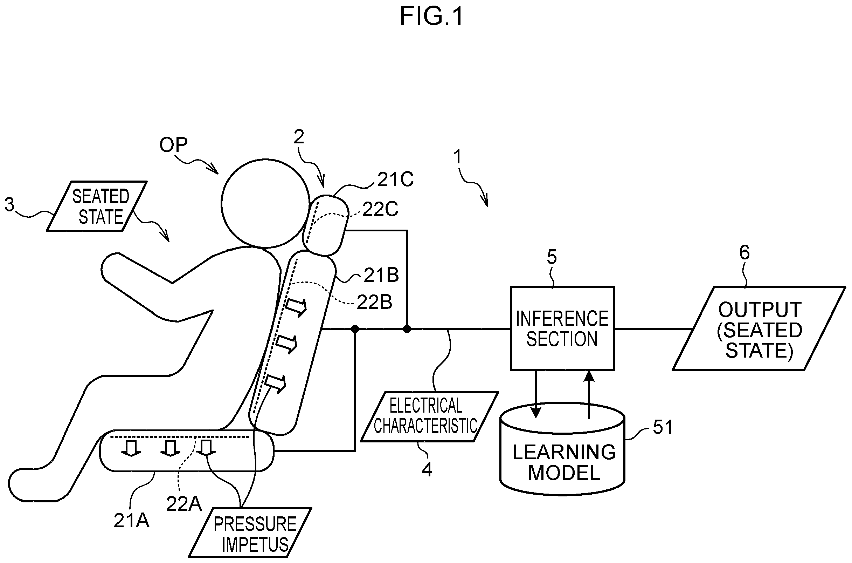

Fig. 1 illustrates an example of a configuration of a posture state inference device 1 serving as an inference device of the present disclosure.

Inference processing in the posture state inference device 1 employs a pre-trained learning model 51 to infer and output a seated state of the occupant related to a posture or movement of the occupant seated on a vehicle seat 2 as an unknown posture state of the occupant OP seated on the vehicle seat. This accordingly enables the posture state of the occupant seated on the vehicle seat 2 to be identified without employing a specialized device or a bulky device and without directly measuring deformation of the soft material contained in the vehicle seat 2. The learning model 51 is trained by using, as input, posture states of an occupant on the vehicle seat 2 (for example seated state values) as labels and an electrical characteristic of the vehicle seat 2 (namely the electrical resistivity of the conductive urethane disposed on the vehicle seat 2) in these posture states. Training of the learning model 51 is described later.

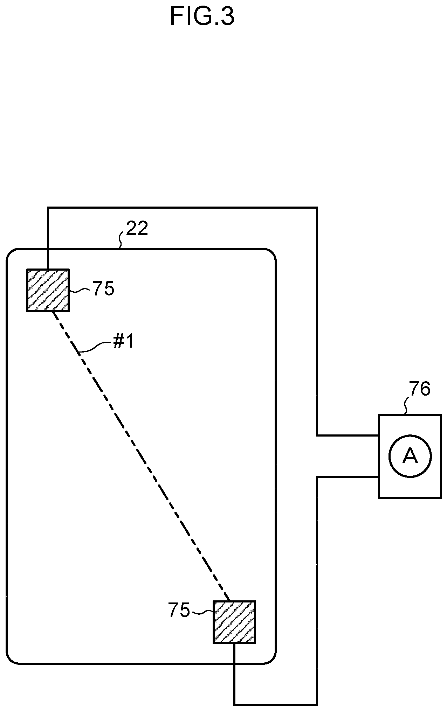

In the present exemplary embodiment, for example, the vehicle seat 2 is configured including a seat cushion 21A, a seatback 21B, and a headrest 21C. A configuration is adopted in which a conductive urethane 22A is disposed on the seat cushion 21A, a conductive urethane 22B is disposed on the seatback 21B, and a conductive urethane 22C is disposed on the headrest 21C. The conductive urethanes 22A, 22B, 22C are connected to an electrical characteristic detection section 76 (see Fig. 3). Note that the conductive urethanes 22A, 22B, 22C are configured from the same conductive urethane. The seat cushion 21A, the seatback 21B, and the headrest 21C will be referred to below by seat 21, as if they were a single body. Moreover, the conductive urethanes 22A, 22B, 22C will be referred to below by conductive urethane 22, as if they were a single body. Note that the conductive urethane 22 is assumed to be formed by any method of filling or impregnating a conductive material, however impregnating achieves a higher conductivity than filling and so is preferable.

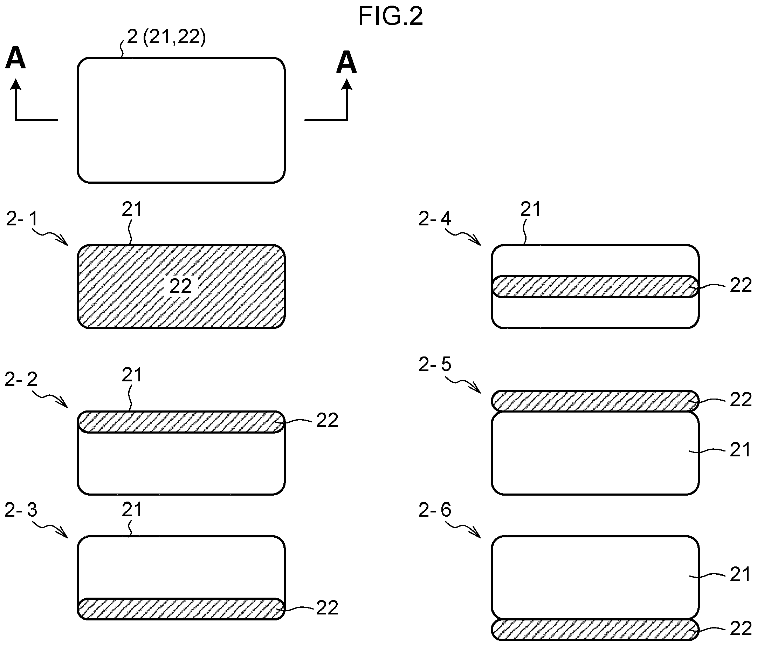

The vehicle seat 2 configured by the seat 21 disposed with the conductive urethane 22 functions as a detection section. As illustrated in Fig. 2, the conductive urethane 22 may be disposed at least on one part of the seat 21, and may be disposed internally or may be disposed externally. Note that for ease of explanation the vehicle seat 2 is represented in Fig. 2 with simple flat plane shapes. As a more specific example, a cross-section A-A of the vehicle seat 2 may be configured with the conductive urethane 22 inside the entire seat 21 as illustrated by seat cross-section 2-1. Moreover, the conductive urethane 22 may be formed inside the seat 21 on the occupant side (front-face side) as illustrated by seat cross-section 2-2, and the conductive urethane 22 may be formed inside the seat 21 on the opposite side to the occupant side (the back-face side) as illustrated by seat cross-section 2-3. Furthermore, the conductive urethane 22 may be formed at the inside of the seat 21 as illustrated by seat cross-section 2-4.

Moreover, the conductive urethane 22 may be disposed at the outside on the occupant side (front-face side) of the seat 21 as illustrated in seat cross-section 2-5, and the conductive urethane 22 may be disposed at the outside on the opposite side to the occupant side (back-face side) as illustrated in seat cross-section 2-6. In cases in which the conductive urethane 22 is disposed at the outside of the seat 21, the conductive urethane 22 and the seat 21 may be simply stacked on each other, or the conductive urethane 22 and the seat 21 may be integrated together using an adhesive or the like. Note that even in cases in which the conductive urethane 22 is disposed at the outside of the seat 21, the softness of the seat 21 is not impeded due to the conductive urethane 22 being configured by the urethane member that is conductive.

For ease of explanation, explanation follows regarding an example in which the vehicle seat 2 is formed with the conductive urethane 22 disposed at the outside on the occupant side (front-face side) of the seat 21 (seat cross-section 2-5).

In the present exemplary embodiment, as illustrated in Fig. 3, an electrical characteristic of the conductive urethane 22 (namely, volume resistivity that is an electrical resistivity) can be detected by a signal from plural (two in Fig. 3) detection points 75 disposed separated by a distance from each other. The example illustrated in Fig. 3 indicates a first detection set #1 for detecting electrical resistivity by signals from the plural detection points 75 disposed at diagonally opposed positions separated by a distance on the conductive urethane 22. Note that the arrangement of the plural detection points 75 is not limited to the positions illustrated in Fig. 3, and they may be positioned at any positions that enable the electrical characteristic of the conductive urethane 22 to be detected. Note that the electrical characteristic of the conductive urethane 22 may employ an output when the electrical characteristic detection section 76 for detecting the electrical characteristic (namely, the volume resistivity that is an electrical resistivity) is connected to the detection points 75.

The posture state described above includes a biased state indicating a bias of the occupant on the vehicle seat 2. Such a biased state is, from out of posture states of the occupant, a state indicating bias of the occupant with respect to the vehicle seat 2. For example, an electrical resistivity detected in the vehicle seat 2 configured by the seat 21 equipped with the conductive urethane 22 at least changes before and after imparting a pressure impetus due to deformation of the conductive urethane 22 when the pressure impetus is imparted to the vehicle seat 2. This means that the electrical resistivity changes before and after biasing of the occupant accompanying pressure impetus to the vehicle seat 2. Thus the bias of the occupant with respect to the vehicle seat 2 can be detected by detecting an electrical resistivity time series, namely by detecting changes in electrical resistivity from a state in which a pressure impetus is not being imparted to the vehicle seat 2 (for example, detecting an electrical resistivity exceeding a predetermined threshold). More specifically, a biased state indicating bias of the occupant with respect to the vehicle seat 2 also includes a contact state of contact of the occupant to the vehicle seat 2 accompanying imparting pressure impetus. Thus contact of the occupant against the vehicle seat 2 is detectable by disposing the conductive urethane 22 on the vehicle seat 2. Moreover, the electrical resistivity changes when there is a change in any one out of the position and distribution, or magnitude, of the pressure impetus imparted to the vehicle seat 2. This means that it is not impossible to detect a contact state including a contact position of the occupant with respect to the vehicle seat 2 from the electrical resistivity changed in a time series.

Note that a biased state is a seated state indicating that the occupant is seated on the vehicle seat 2. Namely, a seated state is identifiable from the position and distribution or magnitude of pressure impetus, and a seated state including seating start is not impossible to detect from the electrical resistivity changed in a time series.

In the present exemplary embodiment, a sensor for detecting the pressure impetus from an occupant is the conductive urethane 22 itself, configured including the urethane member serving as a conductive soft material, and so any uncomfortable feeling when sitting can be reduced to a great extent compared to other sensors hitherto. This means that there is no impairment to ride comfort during measurement, thereby enabling both measurement and ride comfort evaluation to be performed at the same time. This is an advantage compared to sensors hitherto that separately perform measurement and ride comfort evaluation, namely there is a great benefit to measurement and evaluation for a prolonged period of time to follow time series changes.

Note that more detection points than the detection points (two) illustrated in Fig. 3 may be employed in order to improve detection accuracy of the electrical characteristic of the conductive urethane 22.

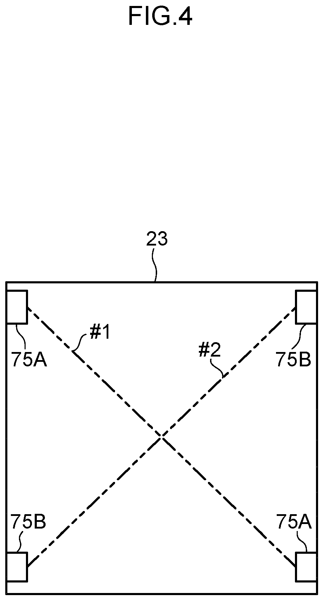

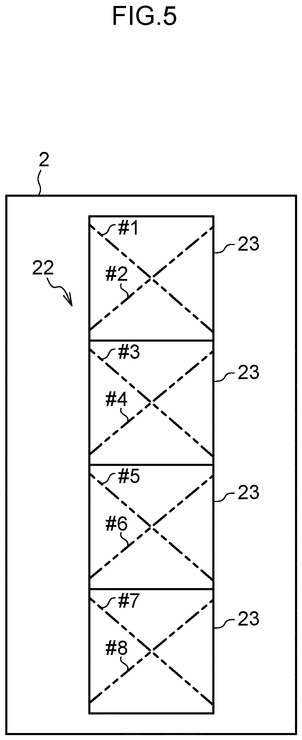

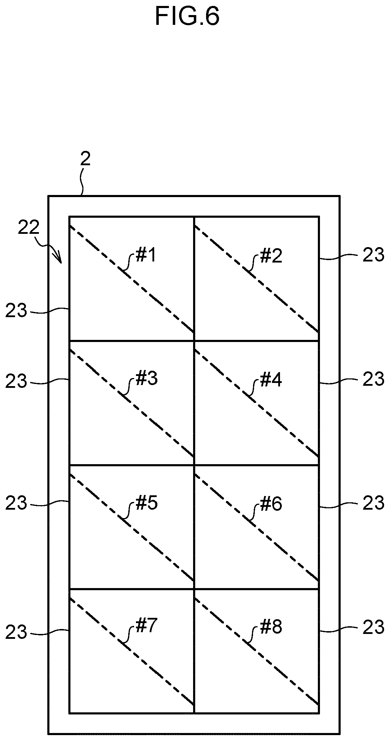

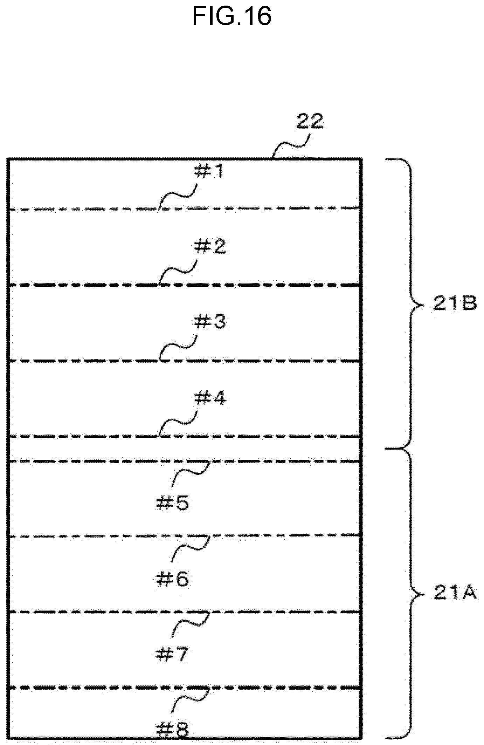

As an example, the conductive urethane 22 may be formed with a single row or plural parallel rows of detection points configured from plural respective conductive urethane pieces disposed in a row, and the electrical characteristic may be detected at each of the plural conductive urethane pieces. For example, conductive urethane pieces 23 (Fig. 4) may configured in an array of the conductive urethane 22 (Fig. 5, Fig. 6). The example illustrated in Fig. 4 illustrates a first detection set #1 for detecting the electrical resistivity from a signal from detection points 75A arranged at diagonally opposed positions separated by a distance, and a second detection set #2 for detecting the electrical resistivity from a signal from detection points 75B arranged at other diagonally opposed positions. Moreover, in the example illustrated in Fig. 5, the conductive urethane pieces 23 (Fig. 4) configure the conductive urethane 22 as an array (4 × 1) along a length direction of the seat 21, and illustrates a sequence configured by the first detection set #1 to an eighth detection set #8. Furthermore, in the example illustrated in Fig. 6, the first detection set #1 is adopted for the conductive urethane pieces 23 (Fig. 4), and the conductive urethane 22 is configured as an array (4 × 2) along the length direction and the width direction of the seat 21, and illustrates a sequence configured by the first detection set #1 to the eighth detection set #8. Moreover, as illustrated in Fig. 16, the seat 21 may be configured using the conductive urethane 22 provided with 8 individual detection sets #1 to #8 spanning from an upper portion to a lower portion of the seatback 21B and from a far side to a nearside of a seat surface of the seat cushion 21A.

Moreover, as another example, detection points may be provided in each sub-divided detection range arising from dividing a detection range on the conductive urethane 22, and the electrical characteristic detected for each of these detection ranges. For example, regions corresponding to the size of the conductive urethane pieces 23 illustrated in Fig. 5 and Fig. 6 may be set as the detection range in the conductive urethane 22, detection points arranged at each of the set detection ranges, and the electrical characteristic detected at each of the detection ranges.

As illustrated in Fig. 1, the posture state inference device 1 includes an inference section 5. A time series of input data 4 representing the magnitude of electrical resistance (electrical resistivity) of the conductive urethane 22 is input to the inference section 5. The input data 4 corresponds to an occupant seated state 3 representing an occupant behavior such as a posture or movement of the occupant seated on the vehicle seat 2. The inference section 5 also outputs, as an inference result, the output data 6 expressing a physical quantity (seated state value) representing a seated state of the occupant seated on the vehicle seat 2. Note that the inference section 5 includes the pre-trained learning model 51.

The learning model 51 is a pre-trained model for deriving a seated state of the occupant, namely a seated state (the output data 6) of the occupant representing the occupant behavior such as a posture or movement of the occupant seated on the vehicle seat 2, from the electrical resistance (the input data 4) of the conductive urethane 22 changed by the pressure impetus according to the occupant seated state 3. The learning model 51 is, for example, a model defining a pre-trained neural network, and is represented by a collection of information of weights (strengths) of connections between nodes (neurons) configuring the neural network.

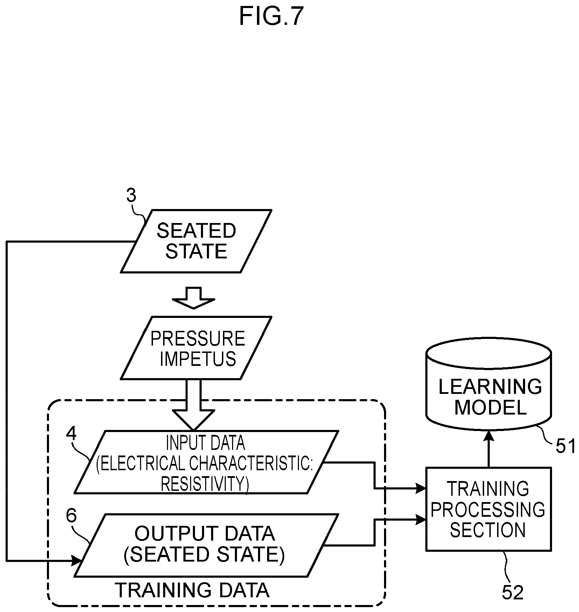

The learning model 51 is generated by training processing of the training processing section 52 (Fig. 7). The training processing section 52 performs training processing using the electrical characteristic (the input data 4) of the conductive urethane 22 that is changed by the pressure impetus arising from the seated state 3 of the occupant. Namely, a large volume of data measured as time series of the electrical resistance in the conductive urethane 22 is employed as the training data, using the occupant seated state 3 as labels. More specifically, the training data includes a large volume of sets configured from input data containing electrical resistivity (the input data 4) and from information (the output data 6) indicating the occupant seated state 3 corresponding to this input data. Time series information is associated with respective electrical resistivities (the input data 4) of the conductive urethane 22 by appending information indicating the measurement time. In such cases, the time series information may be associated by appending information indicating the measurement time to a set of time series electrical resistivity of the conductive urethane 22 for a determined time period as the occupant seated state 3.

Next, description follows regarding the training processing section 52.

In training processing performed by the training processing section 52, the vehicle seat 2 configured by the seat 21 arranged with the conductive urethane 22 as described above may be applied as the detection section, and the occupant seated state 3 and the electrical resistivity (the input data 4) from the conductive urethane 22 may be employed as the training data. For example, an occupant OP is instructed to maintain or to operate in a seated state indicating a specific behavior such as a posture or movement on the vehicle seat 2, and the electrical resistivity when this is performed is detected and associated with the seated state as the training data.

Note that the seated state includes states indicating a time series of occupant behavior. Such states indicating a time series of occupant behavior include static states from occupant postures where movement on the vehicle seat 2 is stable, and dynamic states from plural postures and movements on the vehicle seat 2 that change in a time series. Moreover, the electrical characteristic (namely, the volume resistivity that is an electrical resistivity) may be detected by connecting the electrical characteristic detection section 76 (Fig. 3) to the detection points 75.

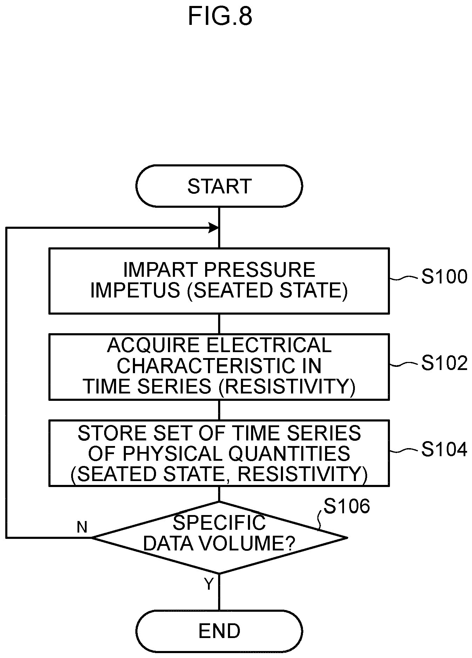

More specifically, the training processing section 52 may be configured including a computer containing a non-illustrated CPU configured so as to execute training data collection processing and training processing. Fig. 8 illustrates an example of the training data collection processing executed by the non-illustrated CPU. At step S 100, the training processing section 52 instructs the occupant OP with a seated state on the vehicle seat 2 (the conductive urethane 22), and at step S102 the electrical resistivity that changes under the pressure impetus according to the seated state is acquired as a time series. Next at step S104, the seated state 3 is appended as a label to the acquired time series of electrical resistivity and then stored. The training processing section 52 repeatedly performs the above processing until there is a predetermined specific number of sets of these seated states 3 of the seated person and of the electrical resistivity of the conductive urethane 22, or until a predetermined specific duration has been reached (negative determination is made at step S 106 until affirmative determination is made). The training processing section 52 is thereby able to acquire and store a time series of electrical resistivity of the conductive urethane 22 for each of the occupant seated states, and training data is configured by the stored sets of a time series of electrical resistivity of the conductive urethane 22 for each of occupant seated states.

However, the seated state (posture) of the occupant OP may be identified by at least one part of a relative positional relationship of each site of the occupant OP with respect to the vehicle seat 2, change or maintenance of each physical quantity such as a distribution, size, or frequency of pressure impetus from each site. Thus features indicating a seated state (posture) of the occupant OP may be thought of being included in a part of these time series of the physical quantity. In the present exemplary embodiment the conductive urethane 22 is employed and the electrical characteristic (volume resistance) reflecting this physical quantity is detectable as a time series.

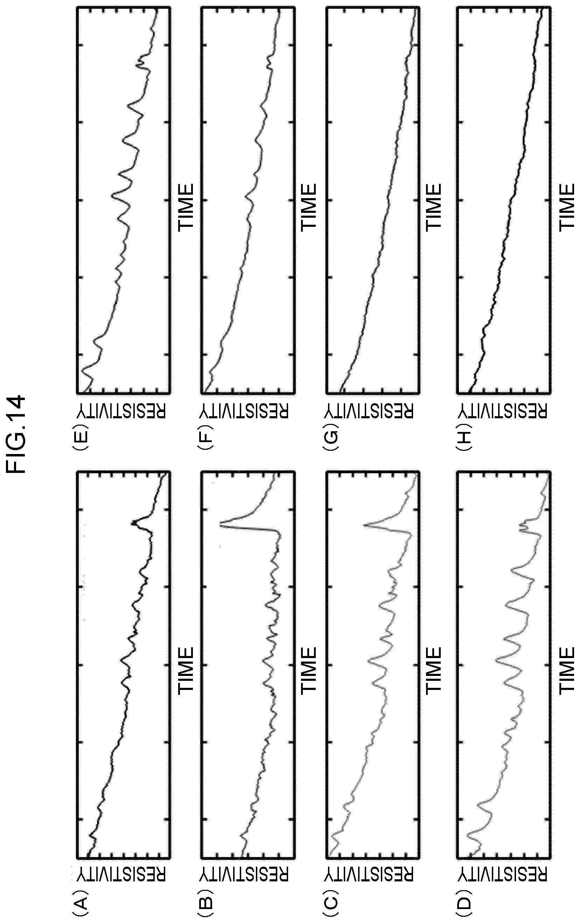

Fig. 14 illustrates an example of an electrical characteristic of the vehicle seat 2 configured by the seat 21 arranged with the conductive urethane 22. Fig. 14 (A) to (H) illustrate electrical characteristics of the vehicle seat 2 configured by the seat 21 arranged with the conductive urethane 22 provided with 8 individual detection sets #1 to #8, as illustrated in Fig. 16. Fig. 14 (A) to (D) indicate the electrical characteristic at each position when the seatback 21B has been divided into four from an upper portion thereof to a lower portion thereof. Moreover, Fig. 14 (E) to (H) indicate the electrical characteristic at each position when the seat cushion 21A has been divided into four from the far side to the nearside of a seating face. Fig. 14 (A) to (H) indicate respective detection results of the detection sets #1 to #8 illustrated in Fig. 16. Moreover, the example illustrated in Fig. 14 illustrates an example of a dynamic state, and so indicates, as the seated state of the occupant, the electrical characteristic in a breathing state of the occupant changed by deep breathing of the occupant.

Each of the electrical characteristics in the occupant seated state illustrated in Fig. 14, namely the electrical characteristics (time characteristic of the electrical resistivity) detected for each of the detection sets #1 to #8 in the seat cushion 21A and the seatback 21B, is a respective feature patterns with respect to a state related to breathing such as deep breathing or the like, as the occupant seated state.

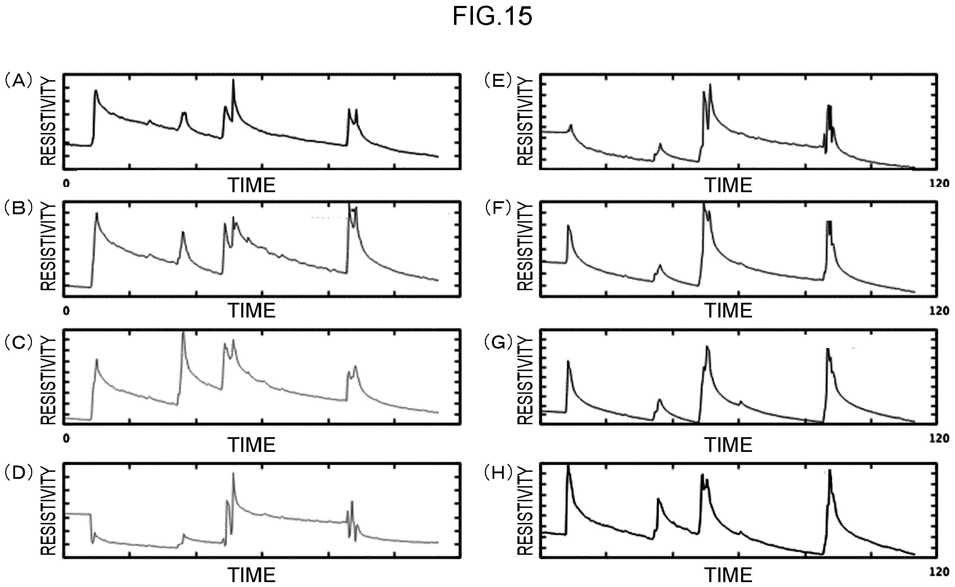

Moreover as the occupant seated state, Fig. 15 indicates the electrical characteristic in states related to a cycle of sitting-style actions from the occupant sitting deeply in the seat cushion 21A, then sitting adjustment so as to sit shallowly therein, and then sitting adjustment so as to again be sitting deeply. Fig. 15 (A) to (H) are detection results for detection sets #1 to #8 indicated in Fig. 16. Note that sitting deeply is when the occupant is sitting at the far side of the seat cushion 21A. Moreover, sitting shallowly is when the occupant is sitting at the nearside of the seat cushion 21A. The respective electrical characteristics detected for each of the detection sets #1 to #8 of the seat cushion 21A and the seatback 21B are feature patterns with respect to states when the occupant performed a cycle of sitting adjustment actions as the seated state. For example, a pattern of detection results detected at the detection set #5 of the seat cushion 21A illustrated at Fig. 15 (E) may be thought of as appearing as a feature pattern. The electrical characteristic when at a time of time T1 may be thought of as corresponding to a distinctive electrical characteristic in an initially seated state. Moreover, the electrical characteristic when at a time of time T2 may be thought of as corresponding to a distinctive electrical characteristic in an state when trying to adjust sitting. Moreover, the electrical characteristic when at a time of time T3 may be thought of as corresponding to a distinctive electrical characteristic in a shallowly seated state. Moreover, the electrical characteristic when at a time of time T4 may be thought of as corresponding to a distinctive electrical characteristic in a state sitting deeply again after sitting adjustment. These feature patterns are also related to patterns of detection results detected by other detection sets. This means that features of the occupant seated state are contained in the electrical characteristic detected in a time series by the conductive urethane 22.

The seated state values indicating the seated states may, for example, be associated with information values expressing a sequence of sitting deeply, trying to re-adjust sitting, sitting shallowly, and sitting deeply again, and may be associated with information values extracted from one part of sitting deeply, trying to re-adjust sitting, sitting shallowly, and sitting deeply again. In cases in which one part is extracted as the seated state, each of the patterns in a corresponding range (patterns of electrical characteristic appearing other than at Fig. 14 (E)) may be associated with the electrical characteristic containing at least a pattern of one part where a feature of the seated state appears (for example, the electrical characteristic at time T3). These feature patterns are thought to correspond to the seated states of the occupant OP on the vehicle seat 2, and function effectively in training processing.

Thus changing the pressure impetus to the vehicle seat 2 by the occupant OP modifying posture according to the instructed seated state and acquiring a time series of the electrical characteristic corresponding to the changing pressure impetus enable time series of electrical characteristic to be associated and stored with the seated person seated states (posture states). Sets of the time series of electrical characteristic and seated state values indicating the instructed seated state configure the training data.

Note that states related to the above sitting-style actions include states related to the vigor when starting to sit, such as sitting forcibly or sitting gently on the vehicle seat 2. Moreover, the above seated states include various states such as states related to breathing such as deep breathing, and other states related to sitting-style actions. For example, the seated states include a state related to reclining of the vehicle seat 2, namely include a state related to an action to modify the angle of the seatback 21B with respect to the seat cushion 21A. These seated states are, similarly to the above seated state (posture) of the occupant OP, identifiable from a relative positional relationship of each site of the occupant OP with respect to the vehicle seat 2 and from at least one part of change or maintenance of each physical quantity such as a distribution, magnitude, and frequency of pressure impetus from each site. Thus features indicating the respective seated state (posture) are thought to be included in one part of the time series of physical quantities, with usage of the conductive urethane 22 enabling the electrical characteristic (volume resistance) reflecting the physical quantity of these states to be detected in a time series.

An example of the above training data is illustrated in the following table. Table 1 is an example of static state data in which time series of electrical resistivity data (r) have been associated with seated state values as training data related to seated state. Table 2 is an example of data in which sets of characteristic data (J) indicating time series of electrical resistivity detected for each detection set illustrated in Fig. 15 are associated with seated state values. Features of seated state, namely feature patterns, are included in some of the characteristic data (J) included in these sets. All of the respective characteristic data (J) is employed as training data. For example, plural characteristic data (J) detected by the vehicle seat 2 and seated state values are employed as the training data.

**Table 1**

| Time Series Electrical Resistivity Data | Seated State |
|---|---|
| r11, r12, r13, ..., r1n | sitting deeply |
| r21, r22, r23, ..., r2n | trying to adjust sitting |
| r31, r32, r33, ..., r3n | sitting shallowly |
| r41, r42, r43, ..., r4n | sitting deeply again |
| ... | ... |
| rk1, rk2, rk3, ..., rkn | deep breathing |
| ... | ... |

**Table 2**

| No. | Time series of electrical resistivity data | State |
|---|---|---|
| 1 | Detection set #1 characteristic data (J1) | sitting deeply → trying to adjust sitting → sitting shallowly → sitting deeply again |
| | Detection set #2 characteristic data (J2) | |
| | Detection set #3 characteristic data (J3) | |
| | Detection set #4 characteristic data (J4) | |
| | Detection set #5 characteristic data (J5) | |
| | Detection set #6 characteristic data (J6) | |
| | Detection set #7 characteristic data (J7) | |
| | Detection set #8 characteristic data (J8) | |
| ... | ... | ... |

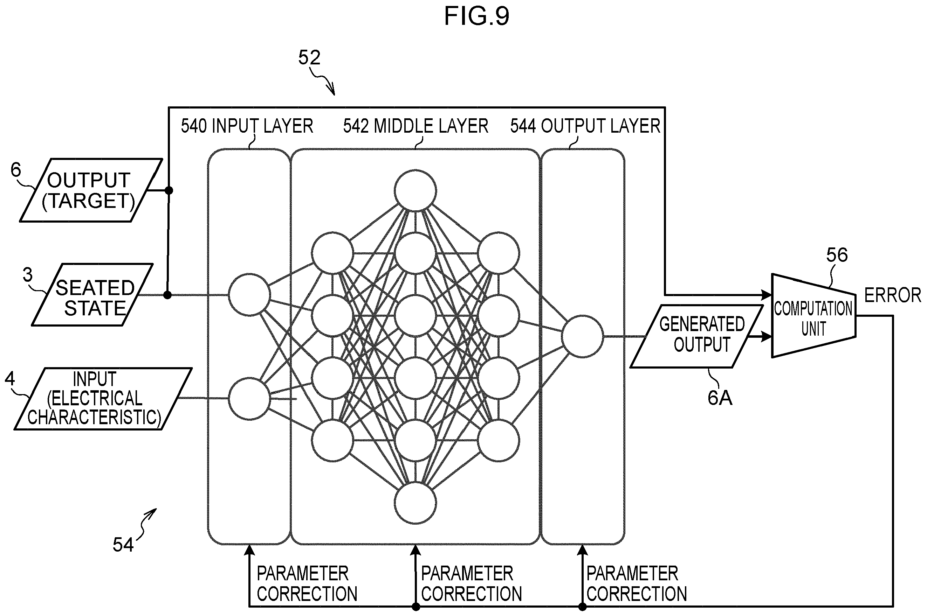

Next, description follows regarding training processing in the training processing section 52. Fig. 9 is a diagram illustrating a function of the non-illustrated CPU of the training processing section 52 in training processing.

The non-illustrated CPU of the training processing section 52 includes the functional sections of a generator unit 54 and a computation unit 56. The generator unit 54 includes a function to generate an output considering a precedence relationship of input that is the electrical resistivity acquired in a time series.

Moreover, the training processing section 52 holds, as the training data, multiple sets of the above input data 4 (electrical resistivity) and the output data 6 that is the occupant seated states 3 when pressure impetus has been imparted to the conductive urethane 22.

The generator unit 54 is configured by a known neural network (NN) including an input layer 540, a middle layer 542, and an output layer 544. Although detailed explanation will be omitted of the known technology of a neural network itself, the middle layer 542 includes multiple node groups (neuron groups) including inter-node connections and feedback connections. Data is input from the input layer 540 to the middle layer 542, and data of computation results of the middle layer 542 is output to the output layer 544.

The generator unit 54 is a neural network for generating generated output data 6A expressing the occupant seated state from the input data 4 (electrical resistance) that was input. The generated output data 6A is data of the occupant seated state when pressure impetus has been imparted to the conductive urethane 22 as inferred from the input data 4 (electrical resistance). The generator unit 54 generates generated output data indicating a state close to the occupant seated state from the input data 4 (electrical resistance) that was input as a time series. The generator unit 54 is trained using multiple input data 4 (electrical resistances) so as to be able to generate the generated output data 6A close to the occupant seated state when pressure impetus has been imparted to the vehicle seat 2, namely to the conductive urethane 22. Another aspect is that a given electrical characteristic that is the input data 4 input as a time series is captured as a pattern, and by learning this pattern, the generated output data 6A close to the occupant seated state when pressure impetus has been imparted to the vehicle seat 2, namely to the conductive urethane 22, can be generated.

The computation unit 56 is a computation unit that compares the generated output data 6A against the output data 6 of the training data, and computes a comparison result error. The training processing section 52 inputs the generated output data 6A and the output data 6 of the training data to the computation unit 56. In response thereto, the computation unit 56 computes the error between the generated output data 6A and the training-data output data 6, and outputs a signal representing this computation result.

Based on the error computed in the computation unit 56, the training processing section 52 tunes weight parameters of the inter-node connections so as to perform training of the generator 54. More specifically, a method such as, for example, gradient descent, error backpropagation, or the like is employed to feedback the respective weight parameters of inter-node connections between the input layer 540 and the middle layer 542 of the generator 54, weight parameters of inter-node connections within the middle layer 542, and weight parameters of inter-node connections between the middle layer 542 and the output layer 544, to the generator 54. Namely, using the training-data output data 6 as the target, all the inter-node connections are optimized so as to minimize error between the generated output data 6A and the training-data output data 6.

The learning model 51 is generated by the training processing of the training processing section 52. The learning model 51 is represented by a collection of information of the weight parameters (weights or strengths) of inter-node connections resulting from training by the training processing section 52.

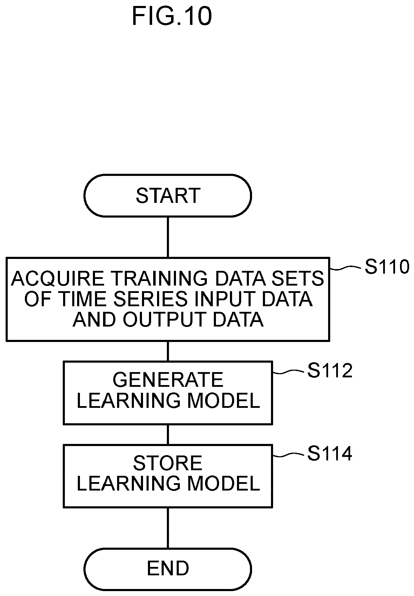

Fig. 10 illustrates an example of a flow of training processing. At step S110, the training processing section 52 acquires the input data 4 (electrical resistance) that is the training data of results measured in time series labeled with information indicating the respective seated state of the occupant OP. At step S112, the training processing section 52 generates the learning model 51 using the time series measurement results as the training data. Namely, a collection of information is obtained for the weight parameters (weights or strengths) of inter-node connections of the training result as trained using the multiple training data as described. above. Then at step S114, data expressed as a collection of information of the weight parameters (weights or strengths) of inter-node connections of the training result is stored as the learning model 51.

Note that the generator unit 54 may use a recurrent neural network including a function to generate output considering the precedence relationship of time series input, and may use another method.

Then in the posture state inference device 1, the pre-trained generator unit 54 generated by a method given above (namely, data represented as a collection of information of weight parameters of inter-node connections of training results) is employed as the learning model 51. As long as a learning model 51 that has been sufficiently trained is employed, it is not impossible to identify the occupant seated state from the time series of electrical resistivity of the vehicle seat 2, namely of the conductive urethane 22.

Note that the processing by the training processing section 52 is an example of processing of a learning model generation device of the present disclosure. Moreover, the posture state inference device 1 is an example of an inference section and an inference device of the present disclosure. The output data 6 that is information indicating the seated state 3 is an example of posture state information of the present disclosure.

However, as described above, the conductive urethane 22 exhibits behavior such as the electrical pathways described above linking in a complex manner, with the electrical pathways elongating/compressing, expanding/contracting, temporarily breaking, and forming new connections according to deformation, with this resulting in a behavior in which the electrical characteristic differs according to the imparted force (for example the pressure impetus). The conductive urethane 22 may accordingly be treated as a reservoir to hold data related to the deformation of the conductive urethane 22. Namely, the posture state inference device 1 may employ the conductive urethane 22 as a network model (hereafter referred to as a PRCN) in so-called physical reservoir computing (PRC). The technology of PRC and PRCN is known, and so detailed explanation thereof will be omitted, however PRC and PRCN are appropriately applied to infer information related to deformation of the conductive urethane 22.

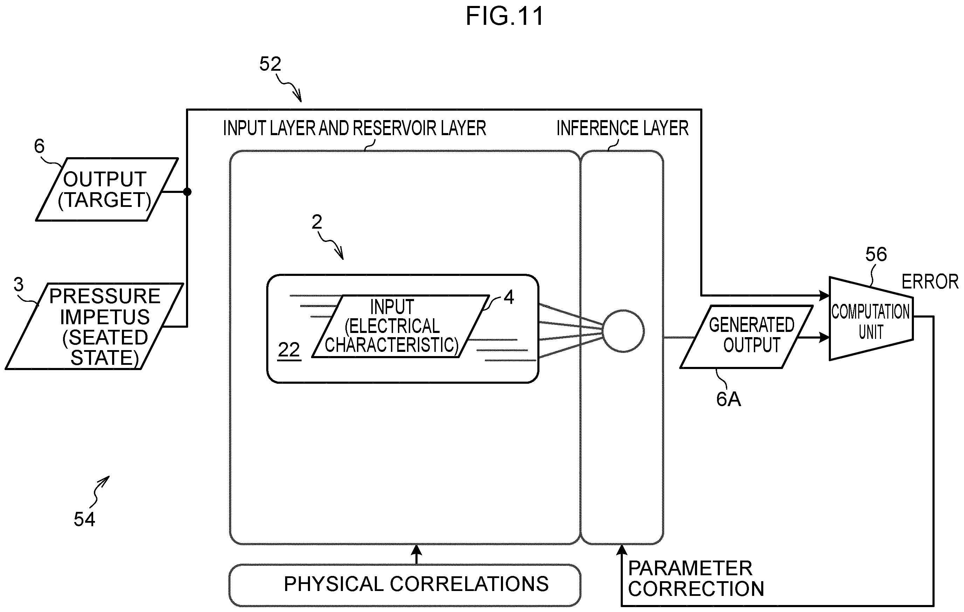

Fig. 11 illustrates an example of the training processing section 52 for training in which the vehicle seat 2 containing the conductive urethane 22 is treated as a reservoir for holding data related to deformation of the vehicle seat 2 containing the conductive urethane 22. The conductive urethane 22 exhibits an electrical characteristic (electrical resistivity) according to each of multiple pressure impetuses, and functions as an input layer for inputting electrical resistivity, or functions as a reservoir layer for holding data related to deformation of the conductive urethane 22. The conductive urethane 22 outputs the electrical characteristic (the input data 4) that differs according to the pressure impetus imparted by the occupant seated state 3, and an imparted pressure impetus 3 (shape of pressed member) can be inferred in an inference layer from the electrical resistivity of the conductive urethane 22. The inference layer may accordingly be trained in the training processing.

The above posture state inference device 1 is, for example, implementable by causing a program representing each of the above functions to be executed by a computer.

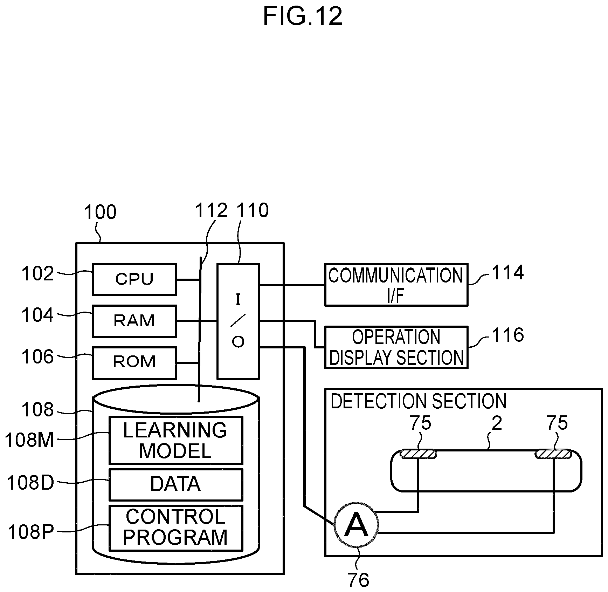

Fig. 12 illustrates an example of a case configured including a computer as the execution device to execute processing to realize the various functions of the posture state inference device 1.

The computer functioning as the posture state inference device 1 includes a computer main body 100 illustrated in Fig. 12. The computer main body 100 includes a CPU 102, RAM 104 such as volatile memory or the like, ROM 106, an auxiliary storage device 108 such as a hard disk device (HDD), and an input-output interface (I/O) 110. The CPU 102, the RAM 104, the ROM 106, the auxiliary storage device 108, and the input-output I/O 110 are configured so as to be connected together through a bus 112 so as to be capable of exchanging data and commands between each other. A communication interface (I/F) 114 for communicating with external devices, and an operation display section 116 such as a display, keyboard, and the like are connected to the input-output I/O 110. The communication I/F 114 has a function to acquire the input data 4 (electrical resistance) between the vehicle seat 2 including the conductive urethane 22. Namely, the communication I/F 114 serves as a detection section included in the vehicle seat 2 arranged with the conductive urethane 22, and is able to acquire the input data 4 (electrical resistance) from the electrical characteristic detection section 76 connected to the detection points 75 in the conductive urethane 22.

A control program 108P to cause the computer main body 100 to function as the posture state inference device 1, serving as an example of the inference device of the present disclosure, is stored in the auxiliary storage device 108. The CPU 102 reads the control program 108P from the auxiliary storage device 108, and expands and executes the processing of the control program 108P in the RAM 104. The computer main body 100 that has executed the control program 108P thereby operates as the posture state inference device 1 serving as an example of the inference device of the present disclosure.

Note that a learning model 108M including the learning model 51, and data 108D including various data, is stored on the auxiliary storage device 108. The control program 108P may be configured so as to be provided by a recording medium such as a CD-ROM or the like.

Next, description follows regarding inference processing in the posture state inference device 1 implemented by a computer.

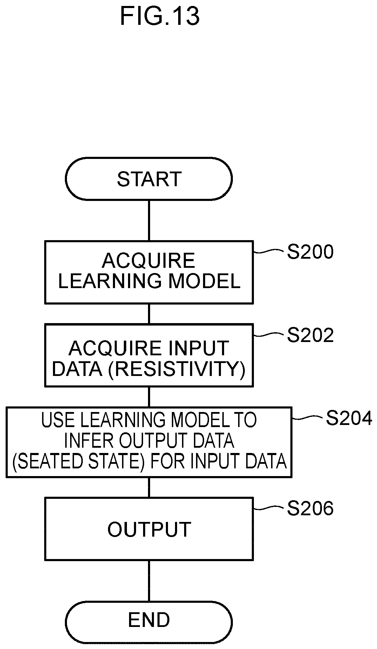

Fig. 13 illustrates an example of a flow of the inference processing by the control program 108P as executed in the computer main body 100.

The inference processing illustrated in Fig. 13 is executed by the CPU 102 when power source of the computer main body 100 is switched ON. Namely, the CPU 102 reads the control program 108P from the auxiliary storage device 108, and expands and executes the processing thereof in the RAM 104.

First at step S200, the CPU 102 acquires the learning model 51 by reading the learning model 51 from the learning model 108M of the auxiliary storage device 108 and expanding the learning model 51 in the RAM 104. More specifically, a network model (see Fig. 9 and Fig. 11) configured by inter-node connections by weight parameters expressed as the learning model 51 is expanded in the RAM 104. The learning model 51 in which the inter-node connections are implemented by weight parameters is thereby built.

Next, at step S202 the CPU 102 acquires, as a time series through the communication I/F 114, the input data 4 (electrical resistance) for an unknown subject for inferring the shape of a pressed member arising from the pressure impetus imparted to the conductive urethane 22.

Next, at step S204, the CPU 102 employs the learning model 51 acquired at step S200 to infer the output data 6 (unknown seated state) corresponding to the input data 4 (electrical resistance) acquired at step S202.

Then at the next step S206, the output data 6 (occupant seated state) of the inference result is output through the communication I/F 114, and the current processing routine is ended.

Note that the inference processing indicated in Fig. 13 is an example of processing executed by the inference method of the present disclosure.

As described above, the present disclosure enables the occupant seated state to be inferred from the input data 4 (electrical resistance) that changes according to the pressure impetus imparted to the conductive urethane 22 by the seated state 3. Namely, the unknown seated state of the occupant can be inferred without using a specialized device or a bulky device and without directly measuring deformation of a soft member.

Moreover, the electrical characteristic from each detection set changes according to the behavior of the occupant OP, and the seated state is reflected in the electrical characteristic (electrical resistance time series), and so the seated state of the occupant is able to be inferred from the electrical resistivity in the conductive urethane 22 as it changes in time series. Namely, even in cases in which there are various seated states, the seated state of the occupant is identifiable by using the above learning model, and this enables the occupant seated state to be inferred.

In the posture state inference device 1 employing the learning model 51 trained by the above training processing, inputting the electrical characteristic of the conductive urethane 22 in various unknown seated states has confirmed that the corresponding seated state of the seated person can be inferred.

As described above, a case of the present disclosure in which the conductive urethane is employed as an example of the soft member has been described, however obviously the soft member is not limited to being a conductive urethane.

Moreover, the technical scope of the present disclosure is not limited to the scope described in the above exemplary embodiment. Various modifications and improvements may be made to the above exemplary embodiment within a range not departing from the spirit thereof, and embodiments with these modifications and improvements are included in the technical scope of the present disclosure.

Moreover, although in the above exemplary embodiment a case of the inference processing and the training processing being implemented by a software configuration by processing has been described using flowcharts, there is no limitation thereto, and for example each processing may be implemented by a hardware configuration.

Moreover, one part of the inference device, for example a neural network such as a learning model or the like, may be configured as a hardware circuit.

### Second Exemplary Embodiment

Description follows regarding a second exemplary embodiment. The second exemplary embodiment is able to draw on the first exemplary embodiment. This means that the following description will be focused on the portions that differ from the first exemplary embodiment, and the same reference numerals will be appended to the same portions as those of the first exemplary embodiment and detailed explanation thereof will be omitted.

In the second exemplary embodiment, the "posture state of an occupant seated on a vehicle seat" is "a posture state in the presence of water of an occupant seated on the vehicle seat". More specifically, the "posture state in the presence of water of an occupant seated on the vehicle seat" encompasses a state of the occupant imparted with moisture by sweating or the like while pressure to deform the soft material is being imparted to the soft material, and includes behavior of an occupant such as posture or movement of the occupant seated on the vehicle seat. The posture state is a seated state in which the occupant is seated on the vehicle seat.

Moreover, in the second exemplary embodiment, the electrical characteristic that changes according to deformation of the soft material is affected by moisture (water content) imparted by sweating or the like of the occupant. A physical quantity (electrical resistivity) expressing an electrical characteristic corresponding to a posture state in the presence of water of the occupant is changed from the physical quantity (electrical resistivity) expressing the electrical characteristic corresponding to a posture state of the occupant in the absence of water. Namely, even if the occupant is in the same posture state, the soft material has a different electrical characteristic according to the water content, enabling the presence or the absence of water, such as sweating of the occupant, to be discriminated.

In the inference device according to the second exemplary embodiment, a pre-trained learning model is employed, and the posture state in the presence of water of an occupant seated on the vehicle seat is inferred from the electrical characteristic of a conductive soft material provided to the vehicle seat. The posture state in the presence of water includes posture states such as, for example, a posture state in the presence of sweat, and a posture state in the presence of water due to at least one out of the body or the clothes of the occupant being damp. The soft material may be disposed on the vehicle seat. The learning model employs, as training data, a time series of the electrical characteristic when pressure and moisture have been imparted to the conductive soft material, and posture state information indicating the posture state in the presence of water of the occupant seated on the vehicle seat when pressure and moisture are imparted to the soft material. The learning model is trained so as, on being input with a time series of the electrical characteristic, to output the posture state information indicating the posture state in the presence of water of the occupant when seated on the vehicle seat corresponding to this time series of the electrical characteristic.

Moreover, in the second exemplary embodiment, a value indicating the pressure impetus in the presence of moisture (hereafter referred to as the moist pressure value) imparted to the vehicle seat is employed as a physical quantity to deform the conductive urethane. The moist pressure value in such cases is generated according to the posture state in the presence of water of the occupant of the vehicle seat, due to sweating or the like. Note that the seated state in the presence of sweat of the occupant seated on the vehicle seat is employed as the posture state in the present exemplary embodiment. Moreover, the electrical resistivity of the conductive urethane is employed as the physical quantity that changes according the pressure impetus in the presence of moisture.

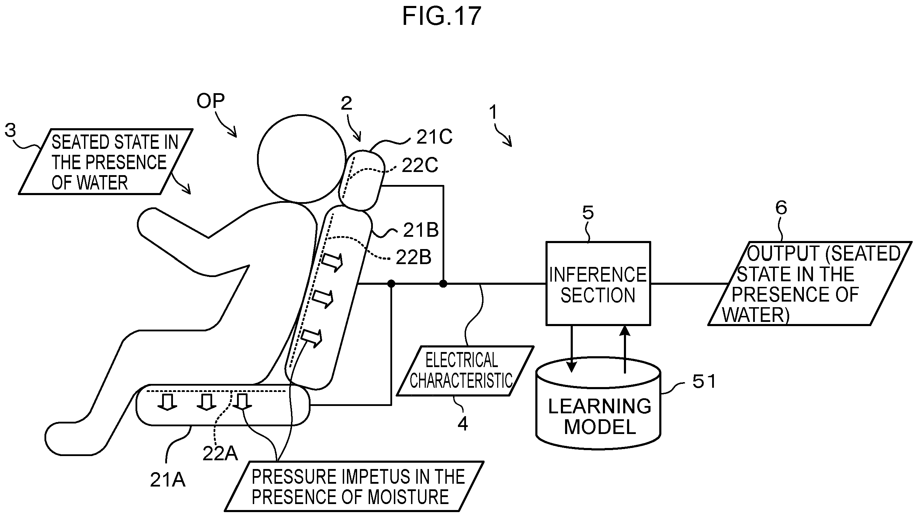

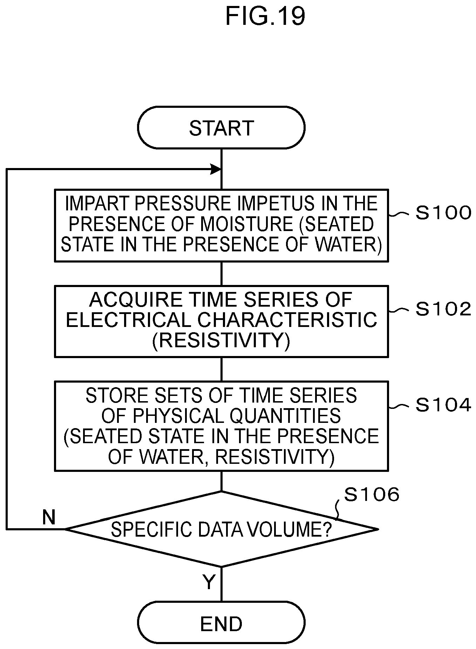

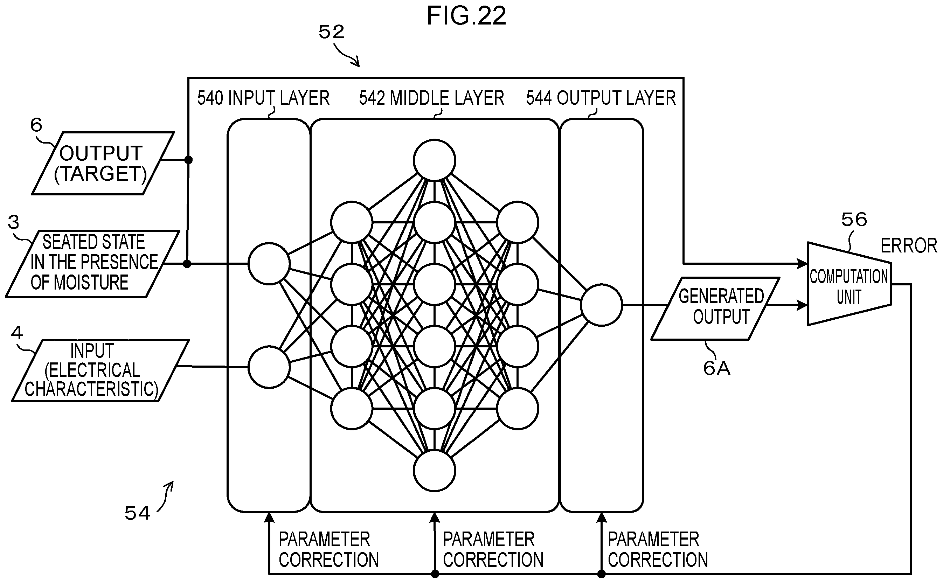

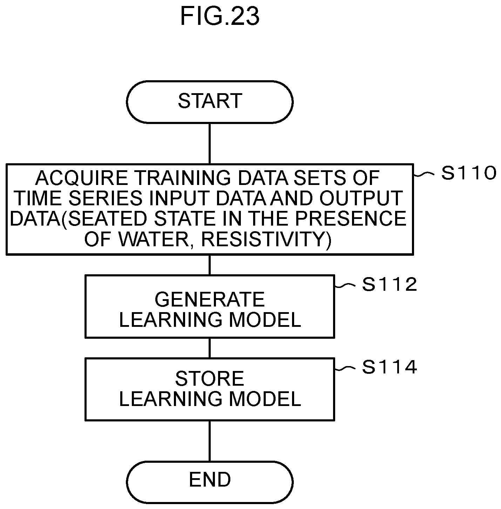

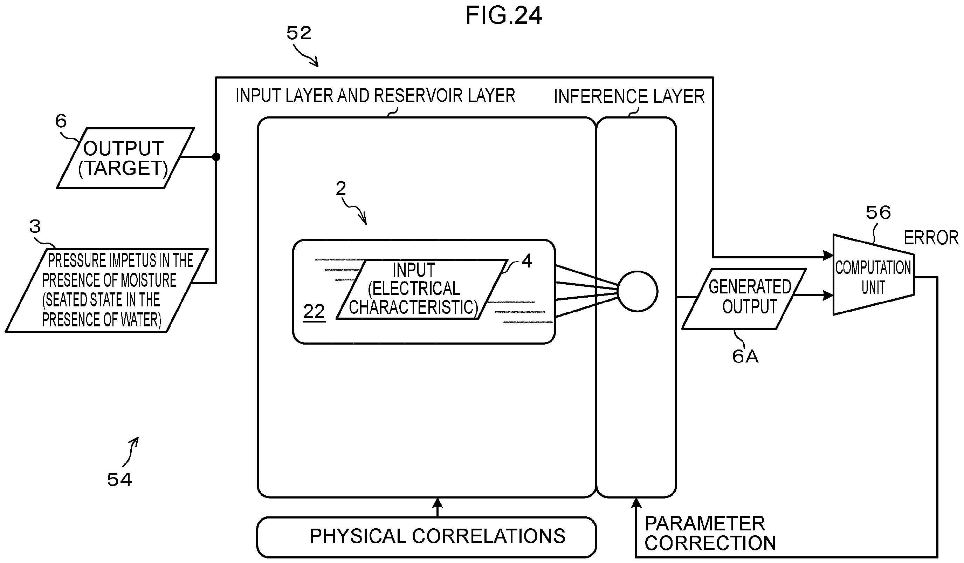

Fig. 17 to Fig. 19, and Fig. 22 to Fig. 25 of the second exemplary embodiment correspond to Fig. 1, Fig. 7 to Fig. 11, and Fig. 13 of the first exemplary embodiment. Fig. 17 to Fig. 19, and Fig. 22 to Fig. 25 can be described by reading "posture state in the presence of water" for the "posture state" in the description of the Fig. 1, Fig. 7 to Fig. 11, and Fig. 13 of the first exemplary embodiment, by reading "the pressure impetus in the presence of moisture" for the "pressure impetus" therein, and by reading "the seated state in the presence of water" for the "seated state" therein.

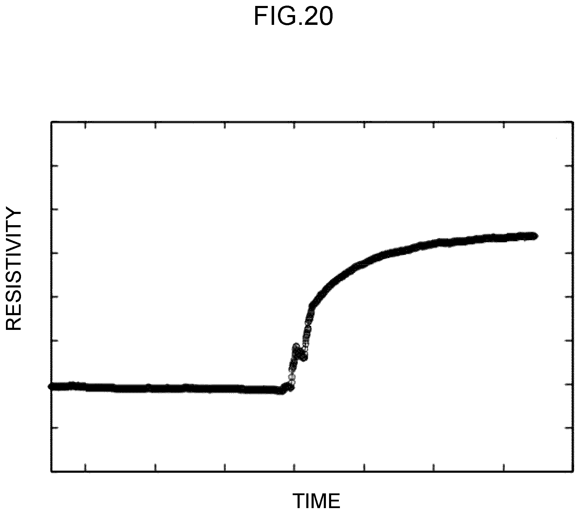

Fig. 20 illustrates an example of an electrical characteristic of the vehicle seat 2 configured by the conductive urethane 22 disposed in the seat 21. The example of Fig. 20 illustrates a way that the electrical characteristic (resistivity) changes in a time series when water is sprayed onto the conductive urethane 22.

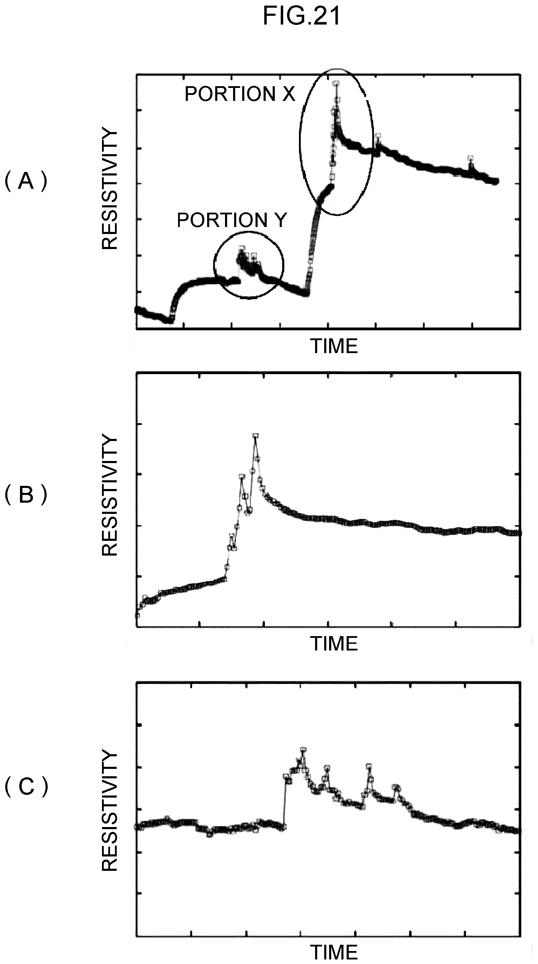

Fig. 21 illustrates different example of the electrical characteristic of the vehicle seat 2 configured by the conductive urethane 22 disposed in the seat 21. The example of Fig. 21 illustrates a way in which the electrical characteristic (resistivity) changes in a time series when water is sprayed onto the conductive urethane 22, similarly to the example of Fig. 20. Fig. 21(B) is an enlarged diagram of portion X of Fig. 21 (A), and Fig. 21 (C) is an enlarged diagram of portion Y of Fig. 21 (A).

As illustrated in Fig. 20 and Fig. 21, the electrical characteristic from the conductive urethane 22 is changed by the water content of the conductive urethane 22, and the possibility of inferring the posture state in the presence of water of the occupant from the electrical resistivity that changes in a time series according to the water content of the conductive urethane 22 can be confirmed even though these are influenced by the water content. Namely, even for various posture states in the presence of water such as sweat, results related to the posture state in the presence of water of the occupant are separable by using the learning model 51 respectively trained therewith, so as to enable discrimination between posture states in the presence of water of the occupant.

The pressure impetus in the presence of moisture to the vehicle seat 2 is changed by the occupant OP modifying posture according to the instructed seated state in the presence of water. Acquiring a time series of electrical characteristic corresponding to these changes in the pressure impetus including moisture thereby enables a time series of electrical characteristic to be associated and stored with the seated state (posture state) in the presence of water of the seated person. Sets configured by these respective time series of electrical characteristic and by respective seated state values indicating the instructed seated state in the presence of water are employed as the training data.

Next, an example of the above training data is illustrated in the following table. Table 3 is an example of training data related to seated state in the presence of water, for data of a static state in which time series of electrical resistivity data (r) are associated with seated state values.

**Table 3**

| Time Series of Electrical Resistivity Data | Seated State |
|---|---|
| r11, r12, r13, ..., r1n | seated state 1 |
| r21, r22, r23, ..., r2n | seated state 2 |
| r31, r32, r33, ..., r3n | seated state 3 |
| r41, r42, r43, ..., r4n | seated state 4 |
| ... | ... |
| rk1, rk2, rk3, ..., rkn | seated state k |
| ... | ... |

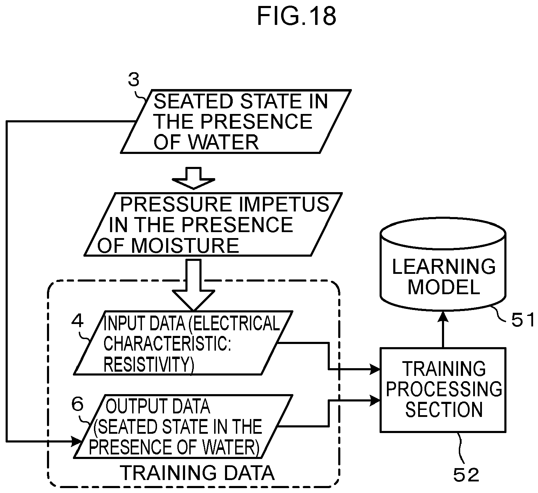

The training processing section 52 illustrated in Fig. 18 executes training processing employing training data such as that described above.

### Third Exemplary Embodiment

Explanation follows regarding a third exemplary embodiment. The third exemplary embodiment is able to draw on the second exemplary embodiment. This means that the following description will be focused on the portions in the third exemplary embodiment that differ from the second exemplary embodiment, and the same reference numerals will be appended to the same portions as those of the second exemplary embodiment and detailed explanation thereof will be omitted.

A hitherto known phenomenon is that an occupant will sweat when experiencing travel sickness. This accordingly enables discrimination as to whether or not an occupant has travel sickness from a level of sweating.

In a control device according to the third exemplary embodiment, a pre-trained learning model is employed to infer the posture state in the presence of water of an occupant seated in a vehicle seat from an electrical characteristic of a conductive soft material provided to the vehicle seat, and information about the inferred posture state is employed to control an environment conditioning device including at least one out of a vehicle seat temperature adjustment device, or an air conditioning device of a vehicle cabin installed with the vehicle seat.

As described in the second exemplary embodiment, posture states in the presence of water include posture states such as, for example, a posture state in the presence of sweat, and a posture state in the presence of water due to at least one out of the body or the clothes of the occupant being damp. Moreover, in the third exemplary embodiment, a posture state accompanying travel sickness is included in the posture state in the presence of sweat.

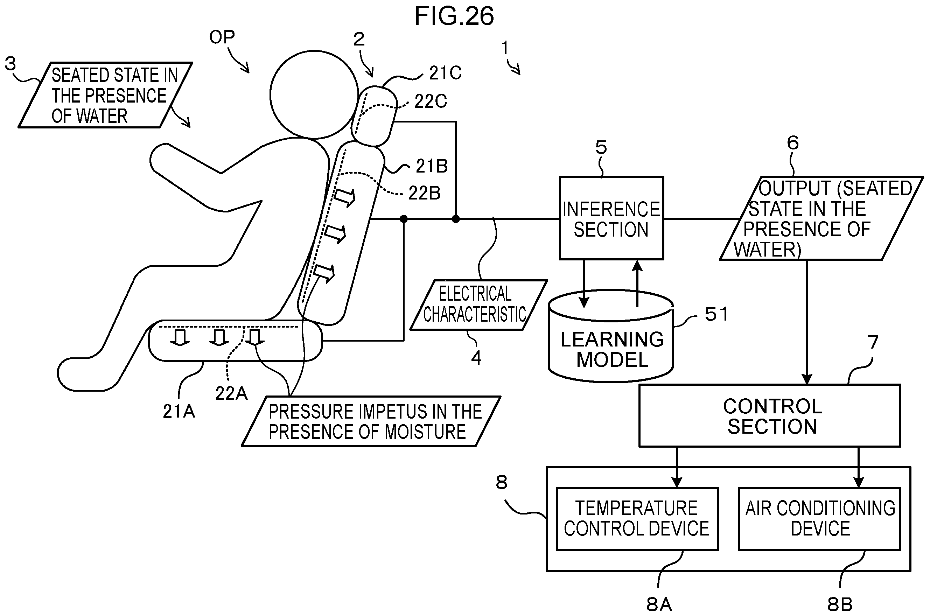

Fig. 26 illustrates an example of a configuration of a control device 10 according to the third exemplary embodiment. The control device 10 has a configuration in which a control section 7 and an environment conditioning device 8 have been added to the inference device 1 illustrated in Fig. 17.

The control section 7 in the control device 10 employs posture state information output by the above inference processing to control the environment conditioning device 8 so as to adjust the environment of the occupant OP. Adjusting the environment of the occupant OP means adjusting at least one out of temperature or humidity in the vicinity of the occupant OP. The environment conditioning device 8 includes a temperature control device 8A of the vehicle seat 2 and an air conditioning device 8B of the vehicle cabin. Note that the environment conditioning device 8 may be configured including any one of the temperature control device 8A or the air conditioning device 8B. The temperature control device 8A is a device to adjust at least one out of temperature or humidity in the vicinity of the occupant OP seated in the vehicle seat 2 and, for example, includes at least one out of a seat cooler for cooling the vehicle seat 2 or a seat heater for warming the vehicle seat 2.

Such a seat cooler is, for example, a variable airflow level fan (omitted in the drawings) that is incorporated into the vehicle seat 2 and that adjusts at least one out of the temperature or humidity in the vicinity of the occupant OP by blowing cooled air toward the occupant OP by rotating the fan. A rise in the temperature of the vehicle seat 2 is suppressed by blowing the cooled air, enabling removal of humid air generated by sweating, for example. Note that the seat cooler may be configured so as to adjust at least one out of temperature or humidity in the vicinity of the occupant OP by rotating the fan in reverse so as to suction air. Moreover, the seat cooler may implemented by circulating a coolant such as water, and may be implemented using another known method.

Such a seat heater is, for example, a fan incorporated into the vehicle seat 2 that adjusts at least one out of temperature or humidity in the vicinity of the occupant OP by blowing warmed air toward the occupant OP. Note that the seat heater may be configured by, for example, incorporating an electric heating cable into the vehicle seat 2 so as to adjust at least one out of temperature or humidity in the vicinity of the occupant OP by heating by flowing current through the electric heating cable so as to warm the vehicle seat 2.

The air conditioning device 8B is a device for conditioning at least one out of temperature or humidity inside the vehicle cabin installed with the vehicle seat 2, and cools or warms the inside of the vehicle cabin by rotating a fan (omitted in the drawings) so as to blow cooled air or warmed air into the vehicle cabin. The air conditioning device 8B includes a dehumidifying function, and is controlled so as to achieve a set temperature or humidity.

In a cases in which the posture state of the occupant OP has been inferred to be a posture state in the presence of sweat, the control section 7 operates, for example, at least one out of the temperature control device 8A or the air conditioning device 8B so as to lower the humidity in the vicinity of the occupant OP. This thereby enables a rise in temperature of the vehicle seat 2 to be suppressed, and humid air generated by sweating is also removed. Namely, sweating in the state of the occupant OP seated in the vehicle seat 2 is suppressed, enabling a comfortable seated state to be obtained.

In cases in which the environment of the occupant OP is adjusted by the temperature control device 8A, for example, a data table may be stored in advance of posture state information in the presence of sweat associated with an airflow level (for example strong, medium, weak, or the like) of the fan of the temperature control device 8A, and the fan of the temperature control device 8A operated at an airflow level according to the inferred posture state in the presence of sweat.

Moreover, in cases in which the environment of the occupant OP is adjusted by the air conditioning device 8B, a data table configured by associating posture state information in the presence of sweat respectively with set temperature and set humidity of the air conditioning device 8B may be stored in advance, and the air conditioning device 8B operated at the set temperature and set humidity according to the inferred posture state in the presence of sweat. For example, in cases in which the posture state of the occupant OP seated on the vehicle seat 2 has been inferred to be a posture state in the presence of sweat, control is performed to actuate the air conditioning device 8B so as to lower the temperature and humidity inside the vehicle cabin. However, the temperature inside the vehicle cabin may be lowered alone in cases in which the temperature inside the vehicle cabin is comparatively high but the humidity is in an appropriate range. Moreover, the humidity inside the vehicle cabin may be lowered alone in cases in which the temperature inside the vehicle cabin is in an appropriate range but the humidity is comparatively high. Such control suppresses sweating during sitting and enables a comfortable seated state to be obtained.

Moreover, in cases in which the inferred posture state in the presence of sweat is a posture state accompanying travel sickness, the control section 7 may control such that a message notifying the travel sickness state is output from a speaker provided to the vehicle.

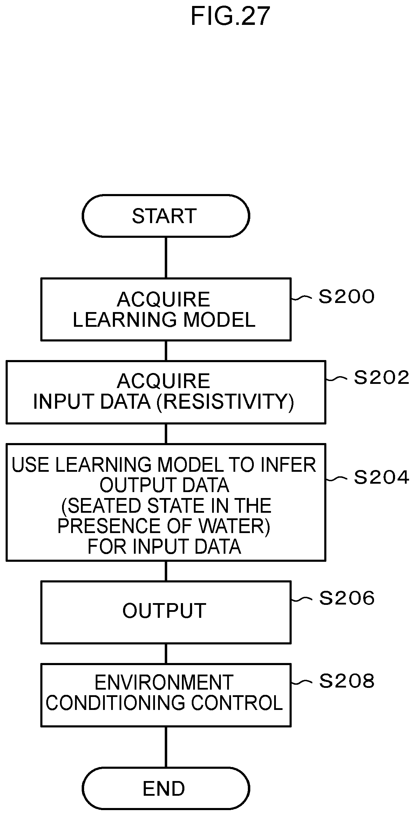

Fig. 27 illustrates an example of a flow of inference/control processing by a control program 108P being executed in the computer main body 100.

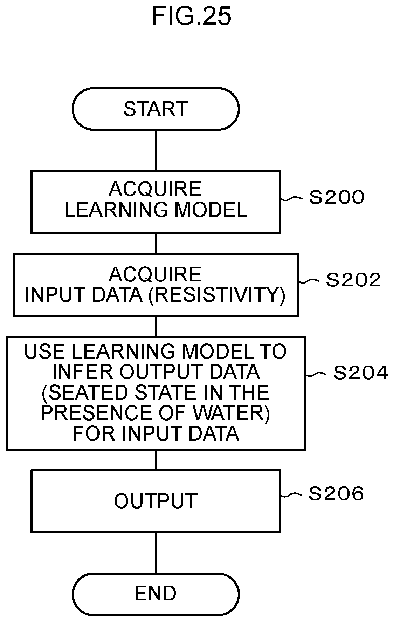

The processing of step S200 to step S204 is the same as the processing of step S200 to step S204 of Fig. 25.

At step S206, the output data 6 of the inference result (the seated state in the presence of water of the occupant) is output to the auxiliary storage device 108.

Then at step S208, the CPU 102 uses the output data 6 of the inference result to execute environment conditioning control to adjust the environment in the vicinity of the occupant OP. Namely, for example in cases in which the posture state of the occupant OP is inferred to be a posture state in the presence of sweat, at least one out of the temperature control device 8A or the air conditioning device 8B is actuated so as to lower the temperature in the vicinity of the occupant OP. More specifically, for example, a fan of the temperature control device 8A is actuated at the airflow level according to the inferred posture state in the presence of sweat. Moreover, the set temperature and the set humidity of the air conditioning device 8B is, for example, set to a lower temperature and a lower humidity than the current temperature and humidity inside the vehicle cabin and the air inside the vehicle cabin is conditioned. Sweating during sitting is suppressed by such environment conditioning control, enabling a comfortable seated state to be obtained. Moreover, in cases in which the inferred posture state in the presence of sweat is a posture state accompanying travel sickness, control is performed so as to output a message notifying the travel sickness state from a speaker provided to the vehicle. This enables, for example, the driver to be induced to pay more attention to driving carefully in consideration of the fact that the occupant is in a travel sick state, thereby reducing travel sickness.

As described above, the third exemplary embodiment enables a seated state in the presence of water of the occupant to be inferred from the input data 4 (electrical resistance) that changes according to the pressure impetus in the presence of moisture imparted to the conductive urethane 22 by the seated state 3 in the presence of water, and enables the environment of the occupant to be adjusted using the inference result. Namely, an unknown seated state in the presence of water of an occupant is inferred without using a specialized device or a bulky device and without directly measuring deformation of a soft member, enabling the environment of the occupant to be adjusted using the inference result.

### Fourth Exemplary Embodiment

Description follows regarding the fourth exemplary embodiment. The fourth exemplary embodiment is able to draw on the first exemplary embodiment. This means that the following description will be focused on the portions of the fourth exemplary embodiment that differ from the first exemplary embodiment, and the same reference numerals will be appended to the same portions as those of the first exemplary embodiment and detailed explanation thereof will be omitted.

In the fourth exemplary embodiment, reference to "a posture state accompanying movement of the occupant seated on a vehicle seat" encompasses a state of the occupant imparting pressure to the soft material to deform the soft material, and includes behavior of the occupant such as a posture, movement, or the like of the occupant seated in the vehicle seat. In the present exemplary embodiment, the posture state will be described for a case in which the posture state is a seated state for an occupant to sit on a vehicle seat. In such cases, "the seated state accompanying movement of the occupant seated on a vehicle seat" encompasses a state related to breathing of the occupant of the vehicle seat, a state related to a sitting-style action of the occupant, and a state related to a reclining operation of an occupant of the vehicle. Moreover, a state related to breathing of the occupant of the vehicle seat includes a state expressing whether or not deep breathing is being performed, a state expressing whether or not yawning is being performed, and the like. Moreover, a state related to a sitting-style action of the occupant of the vehicle seat includes a state expressing a sitting adjustment action such as sitting deeply in the vehicle seat, followed by sitting adjustment to sit shallowly, and then sitting adjustment to sitting deeply again, a state in which the body sways forwards and backwards or left and right, a state related to the vigor of sitting when starting to sit, such as sitting forcibly or sitting gently on the vehicle seat, a state expressing an action to switch pressing of pedals related to driving the vehicle such as an accelerator pedal and a brake pedal, a state expressing an action to cross/uncross left and right legs, a state expressing an action raising one or both left and right thighs, and the like. Moreover, the state related to a reclining action of an occupant of the vehicle includes a state expressing an action movement to switch an angle of the seatback of the vehicle seat.

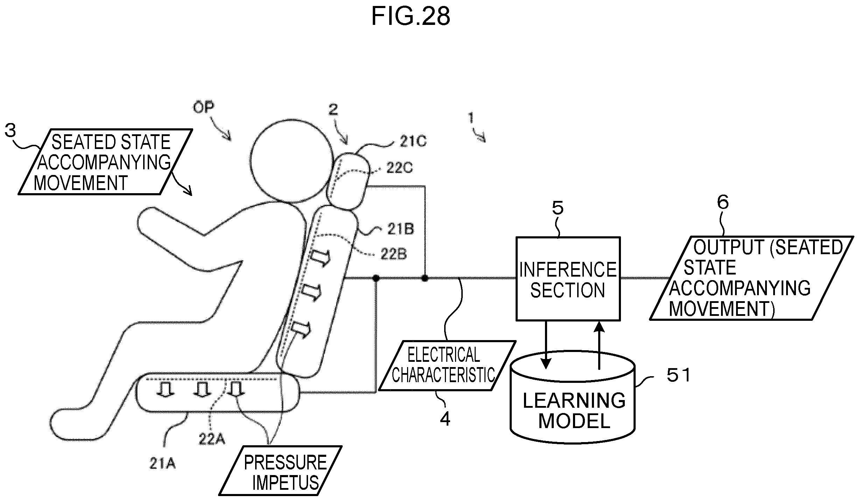

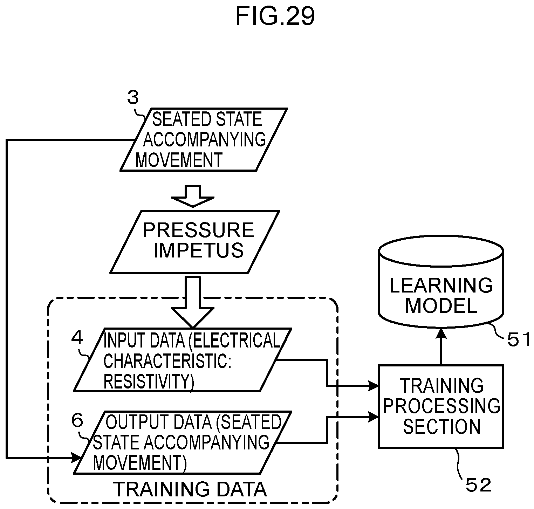

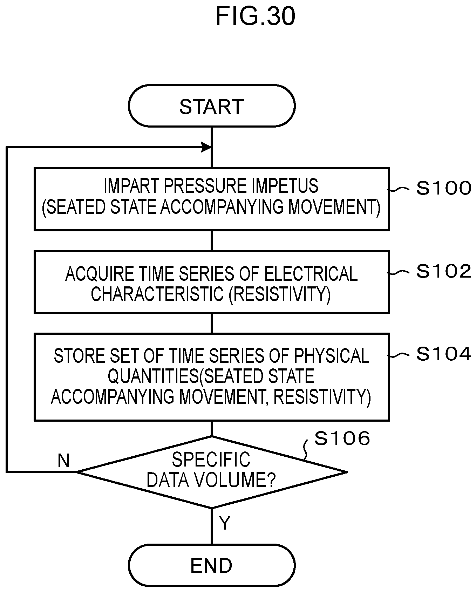

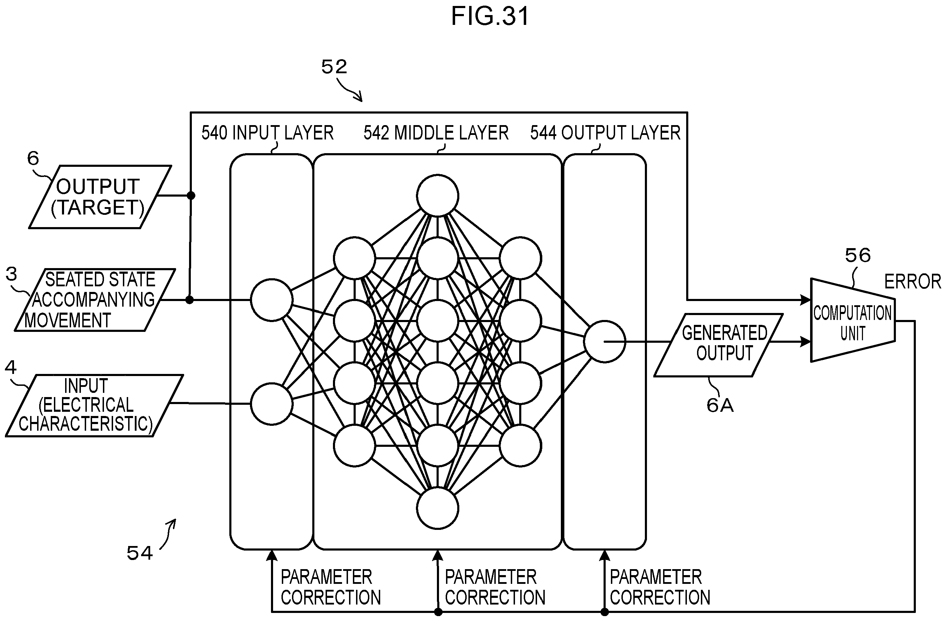

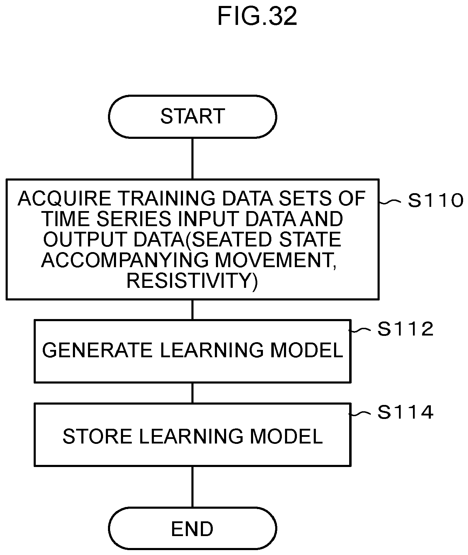

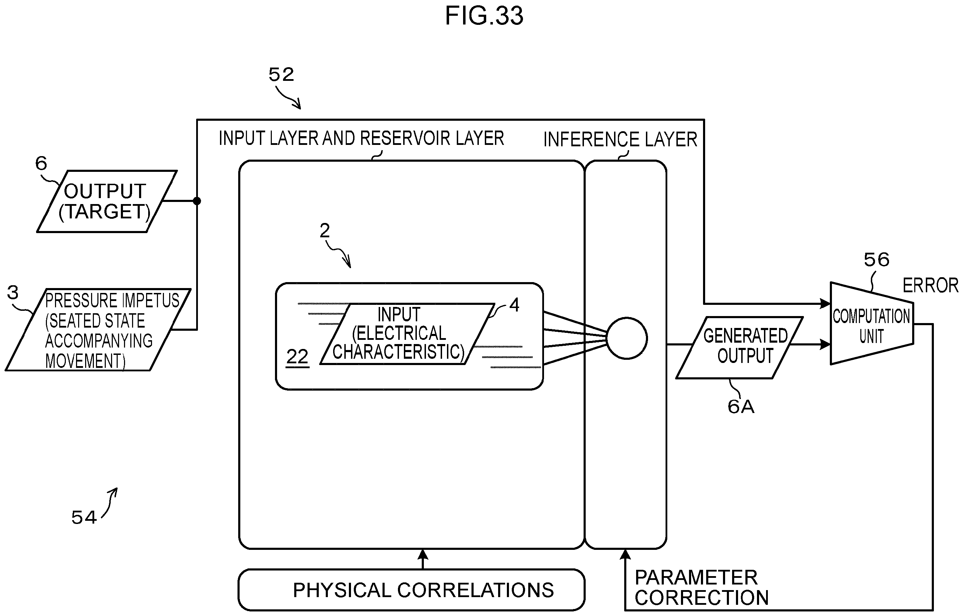

Fig. 28 to Fig. 34 in the fourth exemplary embodiment correspond to Fig. 1, Fig. 7 to Fig. 11, and Fig. 13 of the first exemplary embodiment. Fig. 28 to Fig. 34 can be described by reading "posture state accompanying movement" for the "posture state" in the description of the Fig. 1, Fig. 7 to Fig. 11, and Fig. 13 of the first exemplary embodiment, and by reading "seated state accompanying movement" for the "seated state" therein.

Note that the seated state accompanying movement includes a state indicating a time series of behavior of an occupant. The state indicating a time series of behavior of an occupant includes a dynamic state of plural postures and movements on the vehicle seat 2 that change in a time series. Moreover, the electrical characteristic (namely, the volume resistivity that is an electrical resistivity) may be detected by connecting the electrical characteristic detection section 76 (Fig. 3) to the detection points 75.

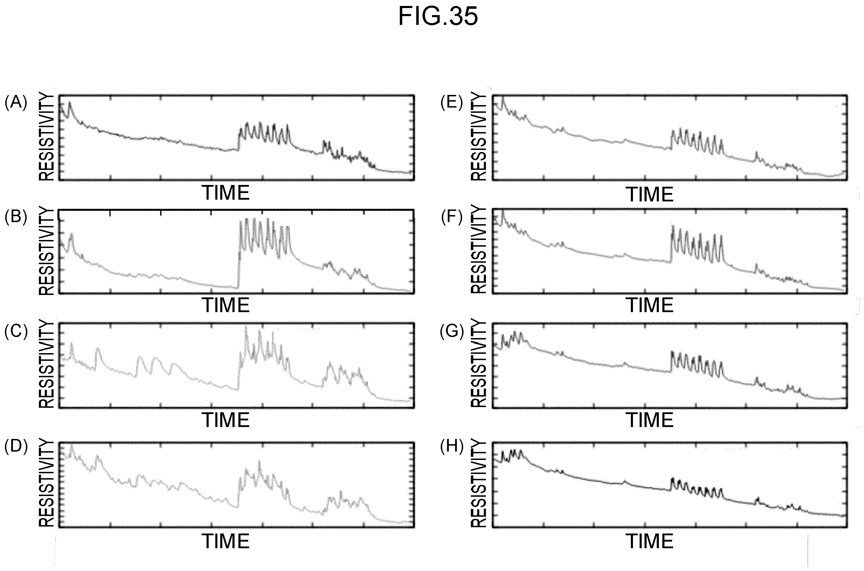

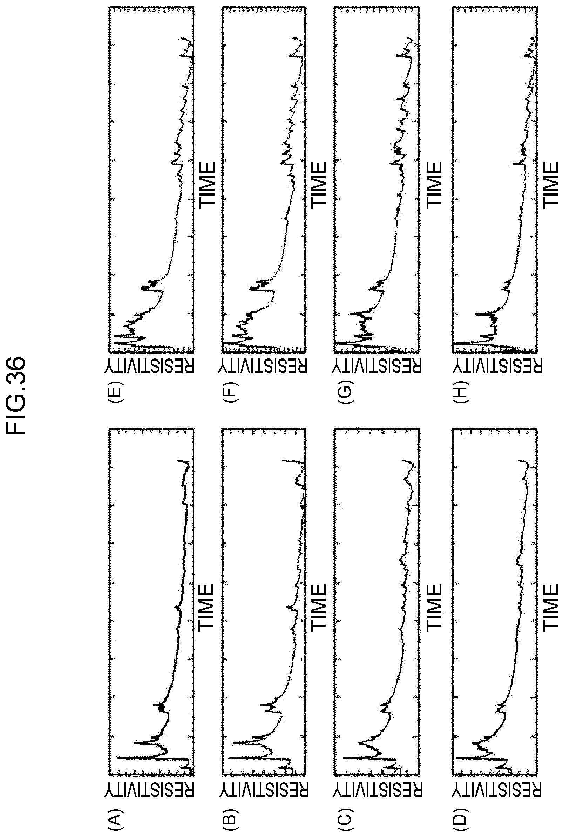

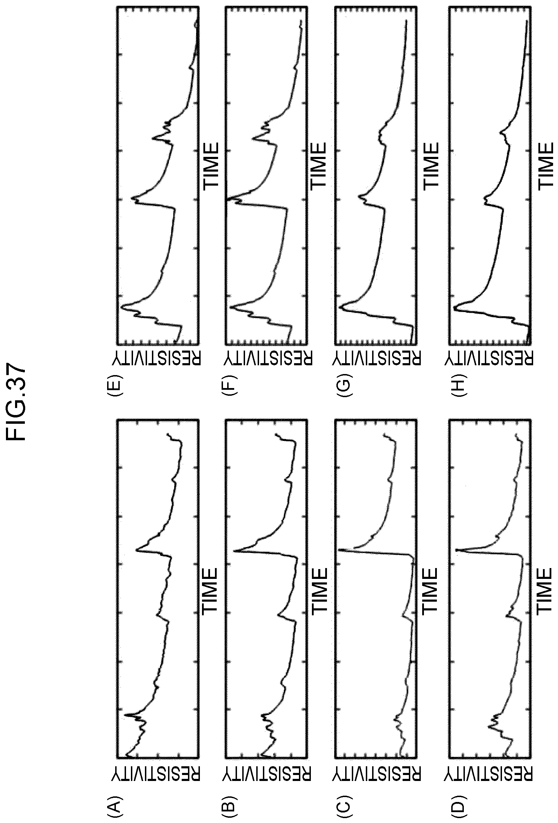

Moreover, the above state related to breathing includes, as well as deep breathing, a state indicating whether or not yawning has been performed. Moreover, the state related to a sitting-style action includes a state related to vigor when starting to sit, such as sitting forcibly or sitting gently on the vehicle seat 2, a state expressing an action to switch pressing pedals related to driving the vehicle, such as an accelerator pedal and a brake pedal, a state expressing an action to cross/uncross left and right legs, and the like. Fig. 35 illustrates an electrical characteristic in a case in which the occupant has yawned as the seated state accompanying movement of the occupant. Moreover, Fig. 36 illustrates an electrical characteristic in a case in which the occupant has performed an action to switch pressing pedals as the seated state accompanying movement of the occupant. Moreover, Fig. 37 illustrates an electrical specific in a case in which the occupant has performed an action to cross/uncross left and right legs. Fig. 35 (A) to (H), Fig. 36 (A) to (H), and Fig. 37 (A) to (H) are respective detection results of the detection sets #1 to #8 illustrated in Fig. 16. Moreover, the seated state accompanying movement described above also includes various other states other than the state related to breathing such as breathing deeply and yawning, and the state related to sitting-style actions. For example, the seated state accompanying movement includes a state related to a reclining action of the occupant of the vehicle seat 2, namely a state related to an action to modify the angle of the seatback 21B with respect to the seat cushion 21A. These seated states accompanying movement are, similarly to the seated state (posture) accompanying movement of the occupant OP described above, able to be identified by at least one part of relative positional relationship of each site of the occupant OP with respect to the vehicle seat 2, and change or maintenance of each physical quantity such as a distribution, magnitude, and frequency of pressure impetus from each site. Thus features indicating the respective seated state (posture) accompanying movement are thought to be included in one part of the time series of physical quantity, and use of the conductive urethane 22 enables the electrical characteristic (volume resistance) that has reflected the physical quantity in these states to be detected in a time series.

The fourth exemplary embodiment enables the seated state accompanying movement of the occupant to be inferred. This accordingly enables a degree of comfort or degree of fatigue, and travel sickness of the occupant to be inferred.

Moreover, as well as changes of the above seated state, when numbness or the like is felt in the bottom or the like due to stagnant blood flow from prolonged sitting, the occupant is urged to get blood flowing by moving their bottom forwards and backwards or left and right so as to eliminate numbness. Moreover, because muscles at the same site fatigue due to the same posture being maintained for a prolonged period of time, not only is there a desire to change the seated state, but also to move the body so as to relax muscle stiffness. In the present exemplary embodiment, such movement of the body of the occupant can be sensed, enabling application to determining a degree of fatigue of the occupant.

### Fifth Exemplary Embodiment

Description follows regarding the fifth exemplary embodiment. The fifth exemplary embodiment is able to draw on the fourth exemplary embodiment. This means that the following description will be focused on the portions in the fifth exemplary embodiment that differ from the fourth exemplary embodiment, and the same reference numerals will be appended to the same portions as those of the fourth exemplary embodiment and detailed explanation thereof will be omitted.

In the fifth exemplary embodiment a "seated state accompanying movement of the occupant on the vehicle seat" encompasses a state related to breathing of the occupant of the vehicle seat, a state related to a sitting-style action of the occupant, and a state related to a change of posture of the occupant of the vehicle seat. Moreover, the state related to a change of posture of the occupant of the vehicle seat includes a state such as a state expressing an action to modify a position of the body in a state of the occupant seated on the vehicle seat.

Note that the state related to breathing of the occupant may be thought of as being closely related to a state related to the mind of the occupant. The state related to the mind of the occupant is called a state expressing at least one out of a degree of fatigue, a degree of tension, or a degree of alertness of the occupant. For example, a quicker speed of breathing is thought to indicate a higher degree of tension of the occupant. In this manner the state related to breathing of the occupant is thought to be closely related to the state related to the mind of the occupant, and so the state related to the mind of the occupant can be inferred when the seated state accompanying movement of the occupant seated in the vehicle seat is inferred to be the state related to breathing of the occupant. For example, by employing table data expressing a correspondence relationship between the state related to breathing of the occupant and the state related to the mind of the occupant, the state related to the mind of the occupant can be identified from the state related to breathing of the occupant.

Moreover, the state related to the sitting-style action of the occupant is thought to be closely related to the state related to the mind of the occupant. For example, a higher frequency of sitting adjustment action is thought to indicate an unsettled occupant and higher degree of tension. In this manner, due to the state related to the sitting-style action of the occupant being thought to be closely related to the state related to the mind of the occupant, the state related to the mind of the occupant can be inferred when the seated state accompanying movement of the occupant seated on the vehicle seat is inferred to be the state related to the sitting-style of the occupant. For example, by employing table data expressing correspondence relationships between states related to the sitting-style of the occupant and states related to the mind of the occupant, the state related to the mind of the occupant can be identified from the state related to the sitting-style of the occupant.

Moreover, the state related to change of posture of the occupant is thought to be closely related to the state related to the mind of the occupant. For example, in cases in which the posture of the occupant hardly changes, the degree of alertness is thought to be low, namely a state close to sleeping. In this manner, due to the state related to change of posture of the occupant being thought to be closely related to the state related to the mind of the occupant, the state related to the mind of the occupant can be inferred when the seated state accompanying movement of the occupant seated on the vehicle seat can be inferred to be the state related to change of posture of the occupant. For example, by employing table data expressing correspondence relationships between the state related to change of posture of the occupant and the state related to the mind of the occupant, the state related to the mind of the occupant can be identified from the state related to the change of posture of the occupant.

As stated above, the "seated state accompanying movement of the occupant of the vehicle seat" in the fifth exemplary embodiment includes a state related to the mind of the occupant.

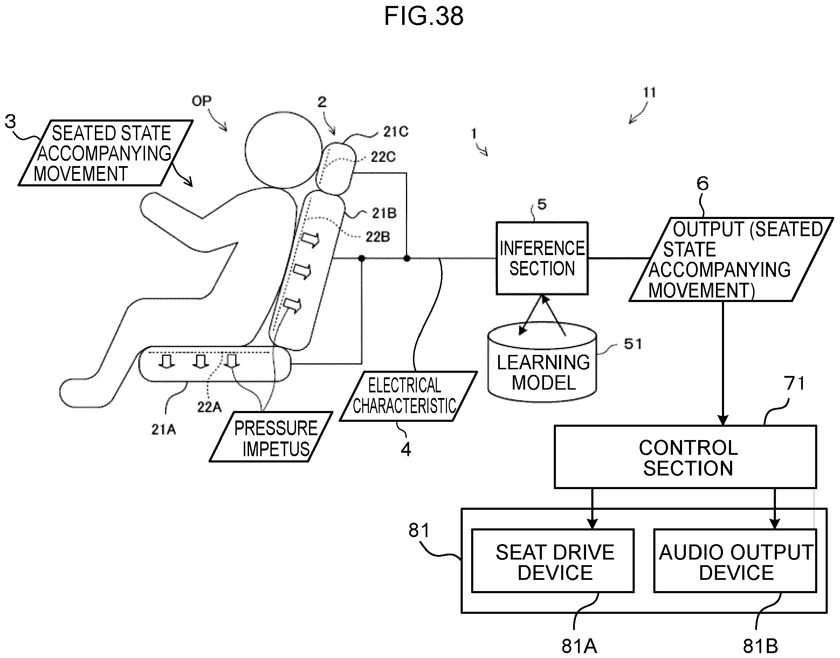

Fig. 38 illustrates an example of a configuration of a control device 11 according to the fifth exemplary embodiment. The control device 11 is a configuration in which a control section 71 and a vehicle device 81 have been added to the inference device 1 illustrated in Fig. 28.

The control section 71 of the control device 11 employs the posture state information output by the inference processing described above to control the vehicle device 81 configuring the vehicle installed with the vehicle seat 2. In the present disclosure the vehicle device 81 includes, as an example, a seat drive device 81A and an audio output device 81B. The seat drive device 81A is a device for performing at least one out of adjusting a position of the seat cushion of the vehicle seat 2 in the front-rear direction with respect to the vehicle, or adjusting an angle of the seatback (reclining angle). The audio output device 81B is a device for notifying the occupant by outputting various audio messages inside the vehicle cabin.

The control section 71 controls the seat drive device 81A such that the position of the vehicle seat 2 is a position according to the inferred state related to the mind of the occupant. For example, when a high degree of fatigue is inferred as the state related to the mind of the occupant, the control section 71 controls the seat drive device 81A so as to make the reclining angle smaller, namely so as to tilt the seatback. This thereby enables the degree of fatigue of the occupant to be lightened. Moreover, when a high degree of fatigue is inferred as the state related to the mind of the occupant, the control section 71 controls so as to output an audio message urging a break from the audio output device 81B.

Moreover for example, when a low degree of alertness, namely a state close to sleeping, has been inferred as the state related to the mind of the occupant, the control section 71 controls the seat drive device 81A so as to increase the reclining angle, namely so as to bring the seatback upright. This thereby enables the degree of alertness of the occupant to be raised. Moreover, in cases in which a low degree of alertness is inferred as the state related to the mind of the occupant, the control section 7 controls to as to output an audio message urging caution from the audio output device 81B. Moreover, in cases in which a low degree of tension, namely a relaxed mood, has been inferred as the state related to the mind of the occupant, the control section 71 may output an audio message urging careful driving from the audio output device 81B.

Moreover, the states related to sitting-style actions on the vehicle seat 2 are, for example, thought to be different for different occupants. Thus the control section 71 may control the seat drive device 81A so as to adjust the position of the vehicle seat 2 according to a state related to a sitting-style action, for example a sitting start action on the vehicle seat 2. This thereby enables the position of the vehicle seat 2 to be adjusted for each occupant, and so for example when the vehicle seat 2 is a driving seat, the position of the vehicle seat 2 may be adjusted automatically when a driver has sat as the occupant on the driving seat, eliminating a need for the driver to adjust the position of the vehicle seat 2 themselves.

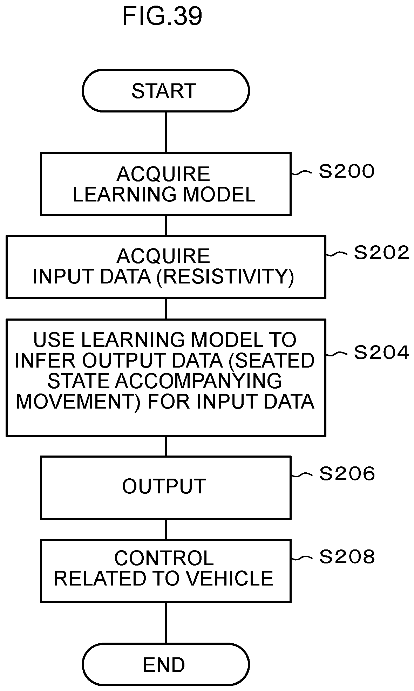

Fig. 39 illustrates an example of a flow of inference/control processing executed by a control program 108P in the computer main body 100.

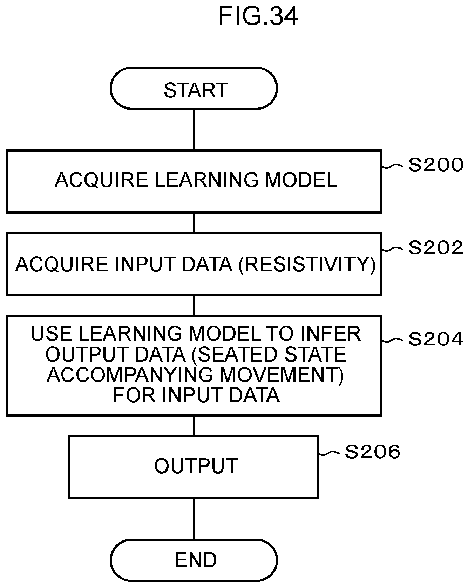

The processing of steps S200 to S204 is the same processing as that of steps S200 to S204 of Fig. 34.

At step S206, the output data 6 of the inference result (the seated state accompanying movement of the occupant) is output to the auxiliary storage device 108.

At step S208 the CPU 102 controls the vehicle device 81 employing the output data 6 of the inference result. For example as described later, the seat drive device 81A is controlled such that the position of the vehicle seat 2 is a position according to the inferred state related to the mind of the occupant. For example, in cases in which a high degree of fatigue is inferred as the state related to the mind of the occupant, the control section 71 controls the seat drive device 81A so as to reduce the reclining angle, namely so as to tilt the seatback. Moreover, for example, in cases in which a high degree of fatigue is inferred as the state related to the mind of the occupant, an audio message urging a break is output from the audio output device 81B. Moreover for example, in cases in which a low degree of alertness, namely a state close to sleeping, has been inferred as the state related to the mind of the occupant, the seat drive device 81A is controlled so as to increase the reclining angle, namely so as to make the seatback upright. Moreover, in cases in which a low degree of alertness is inferred as the state related to the mind of the occupant, for example, an audio message urging caution is output from the audio output device 81B. Moreover, for example, when a low degree of tension, namely a relaxed mood, has been inferred as the state related to the mind of the occupant, an audio message urging careful driving is output from the audio output device 81B.

Note that the inference/control processing illustrated in Fig. 39 is an example of processing executed by a control method of the present disclosure, and various other control may also be performed.

For example, control may be performed such that an airbag device for the occupant of the vehicle seat 2 is switched OFF when inferred that there is no one seated in a vehicle seat 2 other than the driving seat, for example the front passenger seat, and such that the airbag device is switched ON when inferred that someone is seated therein. This thereby enables the airbag device to be prevented from being actuated unnecessarily.

Moreover, the acquired posture state information may be collected and employed for various evaluations. For example, a seated state accompany pedal operation of pedals including at least one out of a brake pedal or an accelerator pedal may be inferred as the seated state accompanying movement, and the posture state information of the inferred seated state accompany pedal operation collected and employed to evaluate driving skill.

Moreover, the posture state information may be collected together for each type of the vehicle seat 2 and employed to evaluate the seat characteristics and the ride comfort for each type of the vehicle seat 2.

Note that the entire content of the disclosures of Japanese Patent Application Nos. 2021-122012, 2021-122013, 2021-122014, 2021-122021, and 2021-122022 are incorporated by reference in the present specification. Moreover, all publications, patent applications and technical standards mentioned in the present specification are incorporated by reference in the present specification to the same extent as if each individual publication, patent application, or technical standard was specifically and individually indicated to be incorporated by reference.

## Claims

1. An inference device comprising:
a detection section that, for a vehicle seat provided with a soft material that is conductive and exhibits a change in an electrical characteristic according to a change in pressure imparted, detects an electrical characteristic between a plurality of predetermined detection points on the soft material of the vehicle seat; and
an inference section that employs, as training data, a time series of the electrical characteristic when pressure has been imparted to the soft material and posture state information indicating a posture state of an occupant of the vehicle seat when imparting pressure to the soft material, that inputs the time series of the electrical characteristic detected by the detection section to a learning model trained to use the time series of the electrical characteristic as input so as to output the posture state information, and that infers posture state information indicating a posture state of the occupant corresponding to the input time series of the electrical characteristic.

2. The inference device of claim 1, wherein:
the electrical characteristic is volume resistivity;
the vehicle seat includes at least one of a seat cushion, a seatback, a headrest, or an armrest;
the posture state includes a seated state of the occupant of the vehicle seat; and
the learning model is trained so as to output, as the posture state information, information indicating a seated state of the occupant corresponding to the detected electrical characteristic.

3. The inference device of claim 2, wherein:
the vehicle seat includes a material imparted with conductivity to at least one part of a urethane member of a structure including a skeleton of at least one of a fiber form or a mesh form or a structure having a plurality of minute air bubbles inside.

4. The inference device of claim 2 or claim 3, wherein:
the seated state includes a state related to breathing of the occupant of the vehicle seat, a state related to a sitting-style action of the occupant, and a state related to reclining of the vehicle seat; and
the learning model is trained so as to output, as the posture state information, information indicating a state corresponding to the detected electrical characteristic that is at least one of the state related to breathing of the occupant, the state related to a sitting-style action of the occupant, or the state related to reclining of the vehicle seat.

5. The inference device of any one of claim 1 to claim 4, wherein:
the learning model includes a model that employs the soft material as a reservoir and that is generated by being trained with a network using reservoir computing using the reservoir.

6. The inference device of any one of claim 1 to claim 5, wherein:
the soft material is a soft material that has conductivity and that has an electrical characteristic that changes according to change in imparted pressure and moisture;
the posture state information is a time series of the electrical characteristic when pressure and moisture is imparted to the soft material, and posture state information indicating a posture state in the presence of water of the occupant of the vehicle seat when imparting pressure and moisture to the soft material; and
the inference section infers posture state information indicating a posture state in the presence of water of the occupant.

7. The inference device of claim 6, wherein:
the posture state in the presence of water of the occupant includes a posture state in the presence of sweat in a seated state of the occupant.

8. The inference device of claim 6 or claim 7, wherein:
the posture state includes a seated state in the presence of water of the occupant of the vehicle seat; and
the learning model is trained so as to output, as the posture state information, information indicating a seated state in the presence of water of the occupant corresponding to the detected electrical characteristic.

9. The inference device of any one of claim 1 to claim 5, wherein:
the posture state information is a time series of the electrical characteristic when pressure is imparted to the soft material and posture state information indicating a posture state accompanying movement of the occupant of the vehicle seat when imparting pressure to the soft material; and
the inference section infers posture state information indicating a posture state accompanying movement of the occupant.

10. The inference device of claim 9, wherein:
the posture state includes a seated state accompanying movement of the occupant on the vehicle seat; and
the learning model is trained so as to output, as the posture state information, information indicating a seated state accompanying movement of the occupant corresponding to the detected electrical characteristic.

11. A control device comprising:
the inference device of any one of claim 6 to claim 8; and
a control section that employs the posture state information inferred by the inference section to control an environment conditioning device including at least one of a temperature adjustment device of the vehicle seat or a conditioning device of a vehicle cabin in which the vehicle seat is installed.

12. The control device of claim 11, wherein:
the vehicle seat includes at least one of a seat cushion, a seatback, a headrest, or an armrest;
the posture state includes a seated state in the presence of water of the occupant of the vehicle seat; and
the control section controls the environment conditioning device using the posture state information inferred by the inference section.

13. A control device comprising:
the inference device of claim 9 or claim 10; and
a control section that controls a vehicle device configuring a vehicle installed with the vehicle seat, using the posture state information inferred by the inference section.

14. The control device of claim 13, wherein:
the inference section infers a state of the occupant as a seated state accompanying movement; and
the control section controls the vehicle device based on the state of the occupant.

15. The control device of claim 14, wherein:
the state of the occupant is a state related to a mind of the occupant, and the control section controls a seat drive device as the vehicle device such that a position of the vehicle seat is a position according to the state related to the mind of the occupant.

16. The control device of claim 14, wherein:
the state of the occupant is a state related to a mind of the occupant, and the control section outputs an audio message according to the state related to the mind of the occupant from an audio output device as the vehicle device.

17. The control device of claim 14, wherein:
the state of the occupant is a state related to a sitting-style action, and the control section controls a seat drive device as the vehicle device such that a position of the vehicle seat is a position according to the sitting-style action.

18. An inference method comprising a computer:
acquiring, for a vehicle seat provided with a soft material that is conductive and exhibits a change in an electrical characteristic according to a change in pressure imparted, an electrical characteristic between a plurality of predetermined detection points on the soft material of the vehicle seat from a detection section that detects the electrical characteristic; and
employing as training data a time series of the electrical characteristic when pressure has been imparted to the soft material and posture state information indicating a posture state of an occupant of the vehicle seat when imparting pressure to the soft material, inputting the time series of the electrical characteristic detected by the detection section to a learning model trained to use the time series of the electrical characteristic as input so as to output the posture state information, and inferring posture state information indicating a posture state of the occupant corresponding to the input time series of the electrical characteristic.

19. An inference program that causes a computer to execute processing, the processing comprising:
acquiring, for a vehicle seat provided with a soft material that is conductive and exhibits a change in an electrical characteristic according to a change in pressure imparted, an electrical characteristic between a plurality of predetermined detection points on the soft material of the vehicle seat from a detection section that detects the electrical characteristic; and
employing as training data a time series of the electrical characteristic when pressure has been imparted to the soft material and posture state information indicating a posture state of an occupant of the vehicle seat when imparting pressure to the soft material, inputting the time series of the electrical characteristic detected by the detection section to a learning model trained to use the time series of the electrical characteristic as input so as to output the posture state information, and inferring posture state information indicating a posture state of the occupant corresponding to the input time series of the electrical characteristic.
